# EUROPÄISCHE PATENTSCHRIFT

(11) **EP 3 190 967 B1**
(45) Veröffentlichungstag und Bekanntmachung des Hinweises auf die Patenterteilung: **28.11.2018**
(21) Anmeldenummer: 15780769.4
(22) Anmeldetag: 09.09.2015
(51) Int. Cl.: A61B 5/113

(54) **VERFAHREN UND VORRICHTUNG ZUR BESTIMMUNG DES ZEITLICHEN VERLAUFS DER ATEMTIEFE**
METHOD AND DEVICE FOR DETERMINING THE TIME CURVE OF THE DEPTH OF BREATH
PROCÉDÉ ET DISPOSITIF DE DÉTERMINATION DE LA VARIATION TEMPORELLE DE LA PROFONDEUR RESPIRATOIRE

(30) Priorität: 10.09.2014 DE 102014218140
(43) Veröffentlichungstag der Anmeldung: 19.07.2017
(73) Patentinhaber: AIT Austrian Institute of Technology GmbH, 1220 Wien (AT)
(72) Erfinder: GARN, Heinrich, A-1220 Wien (AT); KOHN, Bernhard, A-1100 Wien (AT); WIESMEYR, Christoph, A-1020 Wien (AT)
(74) Vertreter: Wildhack & Jellinek
(86) Internationale Anmeldenummer: PCT/EP2015/070608
(87) Internationale Veröffentlichungsnummer: WO 2016/038088

(56) Entgegenhaltungen:
- US-A1- 2014 243 649
- FALIE D ET AL: "Statistical algorithm for detection and screening sleep apnea", SIGNALS, CIRCUITS AND SYSTEMS, 2009. ISSCS 2009. INTERNATIONAL SYMPOSIUM ON, IEEE, PISCATAWAY, NJ, USA, 9. Juli 2009 (2009-07-09), Seiten 1-4, XP031513927, ISBN: 978-1-4244-3785-6
- SCHALLER CHRISTIAN ET AL: "Time-of-flight sensor for respiratory motion gating", MEDICAL PHYSICS, AIP, MELVILLE, NY, US, Bd. 35, Nr. 7, 13. Juni 2008 (2008-06-13), Seiten 3090-3093, XP012116135, ISSN: 0094-2405, DOI: 10.1118/1.2938521
- JOCHEN PENNE ET AL: "Robust real-time 3D respiratory motion detection using time-of-flight cameras", INTERNATIONAL JOURNAL OF COMPUTER ASSISTED RADIOLOGY AND SURGERY, SPRINGER, DE, Bd. 3, 5. Juli 2008 (2008-07-05), Seiten 427-431, XP007908282, ISSN: 1861-6410, DOI: 10.1007/S11548-008-0245-2

## Beschreibung

Die Erfindung betrifft ein Verfahren zur Bestimmung zeitlichen Verlaufs der Atemtiefe einer, insbesondere schlafenden, Person. Weiters betrifft die Erfindung eine Vorrichtung zur Bestimmung des zeitlichen Verlaufs der Atemtiefe. Schließlich betrifft die Erfindung einen Datenträger mit einem Programm zur Bestimmung des zeitlichen Verlaufs der Atemtiefe.

Es werden laufend zu einzelnen Aufnahmezeitpunkten Höhenprofile der Person erstellt. Höhenprofile aus benachbarten Aufnahmezeitpunkten werden zu Segmenten zusammengefasst. Der Bereich, der den Bauch- oder Brustbereich der Person in Abhängigkeit vom jeweiligen Referenzpunkt oder Referenzbereich angibt, wird als Betrachtungsbereich ausgewählt.

Für jedes Höhenprofil innerhalb des Segments wird jeweils gesondert der Mittelwert der Abstände der innerhalb des Betrachtungsbereichs befindlichen Punkte des Höhenprofils zu einem Referenzpunkt oder Referenzgegenstand ermittelt. Für das Segment wird ein Signal ermittelt, dem zum jeweiligen Aufnahmezeitpunkt des Höhenprofils der für dieses Höhenprofil ermittelte Mittelwert zugeordnet wird.

Basierend auf dem ermittelten Signal, insbesondere basierend auf dessen Signalamplitude, werden ein oder mehrere den zeitlichen Verlauf der Atemtiefe charakterisierende Werte ermittelt

Aus dem Stand der Technik ist eine Vielzahl von Verfahren bekannt, mit denen die Atmungsaktivität einer Person, insbesondere während des Schlafs, aufgezeichnet wird. Bei diesen bekannten Verfahren wird die zu untersuchende Person jeweils unmittelbar mit unterschiedlichen Sensoren verbunden, die die Person während des Schlafs stören können. Auch die mitunter zur sofortigen Auswertung der Sensorsignale verwendeten Kabel und Leitungen können den Schlaf der betreffenden Person stören. FALIE D ET AL: "Statistical algorithm for detection and screening sleep apnea", SIGNALS, CIRCUITS AND SYSTEMS, 2009. ISSCS 2009. INTERNATIONAL SYMPOSIUM ON, IEEE, PISCATAWAY, NJ, USA, 9. Juli 2009 (2009-07-09), Seiten 1-4, ISBN: 978-1-4244-3785-6, beschreibt eine Bestimmung des zeitlichen Verlaufs der Atemtiefe einer Person.

Aufgabe der Erfindung ist es daher, ein Verfahren und eine Vorrichtung zur Verfügung zu stellen, die für die zu untersuchende Person wenig störend ist und insbesondere berührungslos die Atmung feststellt.

Die Erfindung löst diese Aufgabe bei dem eingangs genannten Verfahren mit den Merkmalen des Patentanspruchs 1. Die Erfindung löst diese Aufgabe bei der eingangs genannten Vorrichtung mit den Merkmalen des Patentanspruchs 9.

Bei einem Verfahren zur Bestimmung des zeitlichen Verlaufs der Atemtiefe einer, insbesondere schlafenden, Person, sieht die Erfindung vor, dass mit einer auf die Person gerichteten Detektoreinheit laufend zu aufeinander folgenden Aufnahmezeitpunkten jeweils ein Höhenprofil der Person erstellt wird,
a) wobei im Höhenprofil eine Anzahl von zumindest zwei Punkten im Raum festgelegt ist, die auf der Oberfläche der Person oder auf der Oberfläche eines auf oder neben der Person befindlichen Gegenstands liegen,
b) wobei für jeden der Aufnahmezeitpunkte das jeweilige Höhenprofil in einer Datenstruktur abgespeichert und zur Verfügung gehalten wird,
c) wobei eine Anzahl von zu aufeinander folgenden Aufnahmezeitpunkten, insbesondere innerhalb eines Zeitbereichs von 3 bis 20 Sekunden, aufgenommenen Höhenprofilen zu einem Segment zusammengefasst wird,
d) wobei ein Bereich, der den Bauch- oder Brustbereich der Person in Abhängigkeit vom jeweiligen Referenzpunkt oder Referenzbereich angibt als Betrachtungsbereich ausgewählt wird,
e) wobei für jedes Höhenprofil innerhalb des Segments jeweils gesondert der Mittelwert der Abstände der innerhalb des Betrachtungsbereichs befindlichen Punkte des Höhenprofils zu einem Referenzpunkt oder Referenzgegenstand ermittelt wird, und für das Segment ein Signal ermittelt wird, dem zum jeweiligen Aufnahmezeitpunkt des Höhenprofils der für dieses Höhenprofil ermittelte Mittelwert zugeordnet wird, und
f) wobei basierend auf dem ermittelten Signal, insbesondere basierend auf dessen Signalamplitude, ein oder mehrere den zeitlichen Verlauf der Atemtiefe charakterisierende Werte ermittelt wird oder werden.

Durch diese vorteilhaften Maßnahmen wird eine berührungslose Detektion der Atmung einer Person ermöglicht, die die zu untersuchende Person im Schlaf nicht stört. Insbesondere ist es nicht erforderlich, Sensoren am Körper der zu untersuchenden Person zu befestigen.

Eine vorteilhafte und numerisch einfache Ausführungsform der Erfindung sieht vor, dass zur Bestimmung des zeitlichen Verlaufs der Atemtiefe einer, insbesondere schlafenden, Person, wobei mit einer auf die Person gerichteten Detektoreinheit laufend zu aufeinander folgenden Aufnahmezeitpunkten jeweils ein Höhenprofil der Person erstellt wird,
a) wobei das Höhenprofil eine Anzahl von zumindest zwei Abstandswerten zur Festlegung jeweils eines Punktes im Raum aufweist, wobei die einzelnen Abstandswerte jeweils den Abstand des Schnittpunktes eines relativ zur Detektoreinheit vorab festgelegten, insbesondere von der Detektoreinheit ausgehenden, Strahls mit der Oberfläche der Person oder der Oberfläche eines auf der oder neben der Person befindlichen Gegenstands von einem Referenzpunkt oder einer Referenzebene angeben
b) wobei für jeden der Aufnahmezeitpunkte jeweils eine Datenstruktur erstellt wird, die das jeweilige Höhenprofil enthält, wobei sämtliche so erstellte Datenstrukturen jeweils dieselbe Größe aufweisen und jeweils Speicherpositionen für die einzelnen Abstandswerte des Höhenprofils aufweisen,
c) wobei eine Anzahl von zu aufeinander folgenden Aufnahmezeitpunkten, insbesondere innerhalb eines Zeitbereichs von 3 bis 20 Sekunden, aufgenommenen Höhenprofilen zu einem Segment zusammengefasst wird,
d) wobei eine Anzahl von Speicherpositionen der Datenstruktur, in denen Abstandswerte abgespeichert sind, die den Abstand des Bauch- oder Brustbereichs der Person in Abhängigkeit vom jeweiligen Referenzpunkt oder Referenzbereich angeben, als Betrachtungsbereich ausgewählt wird,
e) wobei für jedes Höhenprofil innerhalb eines Segments jeweils gesondert der Mittelwert der innerhalb des Betrachtungsbereichs befindlichen Abstandswerte ermittelt wird, und für das Segment ein Signal ermittelt wird, dem zum jeweiligen Aufnahmezeitpunkt des Höhenprofils der für dieses Höhenprofil ermittelte Mittelwert zugeordnet wird, und
f) wobei basierend auf dem ermittelten Signal, insbesondere basierend auf dessen Signalamplitude, ein oder mehrere den zeitlichen Verlauf der Atemtiefe charakterisierende Werte ermittelt wird oder werden.

Eine alternative vorteilhafte und numerisch präzise Ausführungsform der Erfindung, die die Verwendung von Höhenprofilen in Form von allgemeinen Punktwolken ermöglicht, sieht vor, dass das Höhenprofil eine Punktwolke mit einer Anzahl von zumindest zwei Punkten im Raum beschreibt, die auf der Oberfläche der Person oder auf der Oberfläche eines auf oder neben der Person befindlichen Gegenstands liegen.

Eine vorteilhafte Extraktion der Atmungstiefe aus dem Signal wird erreicht, indem zur Charakterisierung der Atemtiefe in einem Segment aus dem Signal zumindest ein Maximum und zumindest ein Minimum extrahiert werden und zumindest eine Differenz zwischen dem Maximum und dem Minimum als die Atemtiefe charakterisierender Wert für den dem Segment zugeordneten Zeitbereich herangezogen wird.

Eine alternative vorteilhafte Extraktion der Atmungstiefe aus dem Signal wird erreicht, indem das Signal einer spektralen Transformation, insbesondere Fourier-Transformation oder Cosinustransformation oder Wavelet-Transformation, unterworfen wird und innerhalb eines vorgegebenen Frequenzbands, insbesondere von 0,1 Hz bis 1 Hz, nach demjenigen Spektralanteil mit der höchsten Signalenergie gesucht wird und die Signalenergie dieses Spektralanteils zur Charakterisierung der Atemtiefe in diesem Segment herangezogen wird.

Zur rauscharmen Extraktion von Atembewegungen, aus denen letztlich eine besonders gute Extraktion der Atemtiefe möglich ist, kann vorgesehen sein, dass das einem Segment zugeordnete Signal nach seiner Erstellung, insbesondere vor der Bestimmung des die Atemtiefe charakterisierenden Werts, einer Rauschfilterung unterzogen wird, wobei insbesondere
a) Signalanteile mit einer Frequenz von mehr als 0,5 Hz unterdrückt werden, und/oder
b) Gleichanteile unterdrückt werden.

Der Verlauf der Atemtiefe über die Zeit kann vorteilhaft ermittelt werden, indem für eine Anzahl von überlappenden oder nicht überlappenden Segmenten jeweils separat die Atemtiefe ermittelt wird.

Eine vorteilhafte adaptive Anpassung des Betrachtungsbereichs, die regelmäßig mit geringem Aufwand vorgenommen werden kann, sieht vor, dass der Betrachtungsbereich für jedes Segment separat, insbesondere auf Grundlage des für das jeweils vorangehende Segment ermittelten Betrachtungsbereichs, ermittelt wird.

Besonders aussagekräftige Höhenprofile können erstellt werden, indem - das Höhenprofil durch eine zweidimensionale Matrixdatenstruktur umfassend eine Anzahl von Zeilen und Spalten charakterisiert wird,
- ein Anzahl von in Zeilen und Spalten rasterförmig angeordneten Positionen vorgegeben wird, an denen jeweils die Abstandswerte des Höhenprofils bestimmt werden
- die Matrixdatenstruktur ein Raster von derselben Größe und Struktur aufweist, und
- die Matrixdatenstruktur erstellt wird, indem die an den jeweiligen Positionen aufgenommenen Abstandswerte an den den Positionen im Raster entsprechenden Speicherpositionen in der Matrixdatenstruktur abgespeichert und zur Verfügung gehalten werden.

Um eine raschere Verarbeitung der Daten zu ermöglichen, den erforderlichen Speicherplatz zu reduzieren sowie das auftretende Rauschen zu reduzieren, kann vorgesehen sein, dass die Matrixdatenstruktur nach ihrer Erstellung in ihren Abmessungen durch eine reduzierte Matrixdatenstruktur ersetzt wird, wobei für rechteckige, insbesondere die gesamte Matrixdatenstruktur abdeckende, und nicht überlappende Bildbereiche von jeweils gleicher Größe in der Matrixdatenstruktur jeweils ein mittlerer Abstandswert ermittelt wird und dieser mittlere Abstandswert dem bezüglich seiner Lage entsprechenden Bildpunkt der reduzierten Matrixdatenstruktur zugeordnet wird.

Eine vorteilhafte Methode zur Bestimmung des Betrachtungsbereichs sieht vor, dass der Betrachtungsbereich gesetzt wird, indem
a) im Höhenprofil, insbesondere in der Matrixdatenstruktur oder in der Punktwolke, eine Anzahl von möglichen Betrachtungsbereichen vorab vorgegeben werden,
b) für das jeweilige Segment die jeweilige Atemtiefe unter Zugrundelegung jedes der möglichen Betrachtungsbereiche ermittelt wird, und
c) derjenige vorgegebene möglichen Betrachtungsbereich als Betrachtungsbereich herangezogen wird, für den die ermittelte Atemtiefe am größten ist.

Eine zweite vorteilhafte Methode zur Bestimmung des Betrachtungsbereichs, die besonders auf anatomische Gegebenheiten Rücksicht nimmt, sieht vor, dass der Betrachtungsbereich gesetzt wird, indem
a) im Höhenprofil, insbesondere in der Matrixdatenstruktur oder in der Punktwolke, mittels Objekterkennung nach Bereichen gesucht wird oder dass vorab Bereiche ausgewählt werden, die einem menschlichen Kopf und Torso entsprechen, und
b) der den Torso abbildende Bereich des Höhenprofils, insbesondere der Matrixdatenstruktur oder der Punktwolke, oder ein Teilbereich dieses Bereichs als Betrachtungsbereich ausgewählt wird, wobei insbesondere
   - zur Detektion des Verlaufs oder der Atemtiefe der Brustatmung der dem Kopf nahe Teilbereich dieses Bereichs als Betrachtungsbereich ausgewählt wird und/oder
   - zur Detektion des Verlaufs oder der Atemtiefe der Bauchatmung der dem Kopf ferne Teilbereich dieses Bereichs als Betrachtungsbereich ausgewählt wird.

Eine rasche Anpassung des Betrachtungsbereichs kann erreicht werden, indem für jedes zu untersuchende Segment oder für einzelne Höhenprofile eines Segments der Betrachtungsbereich mittels Objekterkennung an die im Segment vorliegenden Höhenprofile ausgehend von der Position des ausgewählten Betrachtungsbereichs im jeweils vorangehenden Segment adaptiv neu ermittelt wird.

Eine dritte vorteilhafte Methode zur Bestimmung des Betrachtungsbereichs, die numerisch stabil und effizient ausführbar ist, sieht vor, dass der Betrachtungsbereich gesetzt wird, indem
a) für sämtliche Punkte des Höhenprofils, insbesondere für alle Einträge der Matrixdatenstruktur oder der Punktwolke, separat die Varianz über das jeweilige Segment bestimmt wird, und
b) ein Bereich oder mehrere Bereiche mit jeweils zusammenhängenden Punkten des Höhenprofils, deren jeweilige Varianz oberhalb eines unteren Schwellenwerts oder innerhalb eines vorgegebenen Intervalls liegen, als Betrachtungsbereich ausgewählt wird oder werden, insbesondere
   - indem zusammenhängende Bereiche von Punkten als Betrachtungsbereich ausgewählt werden, deren Größe eine vorgegebene Anzahl von Punkten übersteigt
   - wobei insbesondere ein Bereich dann als zusammenhängend angesehen wird, wenn jeder Punkt des Bereichs ausgehend von einem anderen Punkt innerhalb dieses Bereichs über jeweils benachbarte Pixel erreichbar ist,
   - wobei vorzugsweise Punkte des Höhenprofils dann als benachbart angesehen werden,
      - wenn sie durch benachbarte Einträge der Matrix-Datenstruktur festgelegt sind oder
      - wenn sie oder ihre Projektion auf eine horizontale Ebene im Raum einen einen Schwellenwert unterschreitenden Abstand voneinander aufweisen.

Um Situationen, bei denen sich die zu untersuchende Person bewegt, detektieren zu können und die in diesen Situationen aufgenommenen fehlerhaften Messwerte verwerfen zu können, kann vorgesehen sein, dass der Betrachtungsbereich in zwei oder mehreren aufeinander folgenden Segmenten separat ermittelt wird und ein Höhenprofil verworfen und eine Fehlermeldung ausgegeben wird, wenn sich der Betrachtungsbereich, insbesondere dessen Größe und/oder dessen Schwerpunkt, gegenüber dem jeweils vorangehenden Segment um einen vorgegebenen Schwellenwert verschiebt.

Zur verbesserten Detektion des Aussetzens der Atmung ist zusätzlich eine akustische Kontrolle vorgesehen. Hierbei ist vorgesehen, dass a) gleichzeitig parallel zur Aufnahme des Abstands der Person von der Detektoreinheit der von der Person abgegebene Schall ermittelt und in Form eines Audio-Signals mit einer Anzahl von Audio-Abtastwerten zur Verfügung gehalten wird,
b) das Audio-Signal in eine Anzahl von Audio-Segmente, insbesondere mit einer Länge von 100 ms bis 1 Sekunde, unterteilt wird und für jedes der Audio-Segmente untersucht wird, ob in diesem menschliche Atemgeräusche oder andere Geräusche enthalten sind, und für jedes Audio-Segment jeweils ein Klassifikationsergebnis zur Verfügung gehalten wird,
c) zeitgleich aufgenommene Audio-Segmente und Segmente einander zugeordnet werden, und
d) nach Audio-Segmenten oder Segmenten mit geringer Atemtiefe oder mit fehlenden Atemgeräuschen gesucht wird und die einem solchen Audio-Segment oder Segment zugeordneten Segmente oder Audio-Segmente ebenfalls auf das Vorliegen geringer Atemtiefe oder fehlender Atemgeräuschen untersucht werden, und
e) für den Fall, dass in einander zugeordneten Segmenten und Audio-Segmenten jeweils geringe Atemtiefe und fehlender Atemgeräusche detektiert wurden, das Fehlen der Atmung der Person festgestellt wird.

Eine besonders bevorzugte Ausführungsform der Erfindung, die eine Detektion des Beginns und des Endes von Atemaussetzern ermöglicht, sieht vor, dass
a) ein Klassifikationssignal erstellt wird, das für jedes Audio-Segment jeweils zumindest ein Klassifikationsergebnis enthält, das die Art und Stärke des jeweiligen Geräuschs im Zeitbereich des jeweiligen Audio-Segment angibt,
b) dass jeweils eine Anzahl von aufeinander folgenden Audio-Segmente zu weiteren Audio-Segmenten, insbesondere mit einer Dauer von 5 bis 10 Sekunden zusammengefasst werden, die vorzugsweise dieselben Zeitbereiche umfassen wie die Segmente,
c) über die innerhalb eines weiteren Audio-Segments enthaltenen Klassifikationsergebnisse eine Mittelung vorgenommen wird und dem weiteren Audio-Segment der jeweilige Mittelwert zugewiesen wird,
d) ein weiteres Klassifikationssignal durch Interpolation der Mittelwerte der weiteren Audio-Segmente erstellt wird,
e) im weiteren Klassifikationssignal sowie im Atemtiefesignal nach Zeitpunkten gesucht wird, zu denen in den beiden Signalen starke Änderungen auftreten,
f) die so erkannten Zeitpunkte werden als umso relevanter eingestuft werden, je stärker die Änderung des jeweiligen Signals zum jeweiligen Zeitpunkt ist,
g) diesen Zeitpunkten jeweils ein diesbezüglicher Relevanzwert zugeordnet wird, und
h) jene Zeitpunkte als Startpunkte oder Endpunkte eines Atemstillstandes detektiert werden, für welche der Betrag des Relevanzwerts über dem Betrag eines Schwellwert liegt, wobei insbesondere
   - der Schwellenwert gebildet wird, indem über einen Zeitbereich, insbesondere vor und/oder nach dem Vergleichszeitpunkt mit dem Referenzmaß ein Mittelwert des Referenzmaßes gebildet wird und der Schwellenwert im Bereich zwischen 120% und 200% dieses Mittelwerts festgelegt wird.

Ein Verfahren zur Durchführung eines erfindungsgemäßen Verfahrens kann vorteilhaft auf einem Datenträger abgespeichert werden.

Die Erfindung betrifft weiters eine Vorrichtung zur Bestimmung des zeitlichen Verlaufs der Atemtiefe einer, insbesondere schlafenden, Person, umfassend
- eine auf eine Schlafgelegenheit, insbesondere ein Bett ausrichtbare Detektoreinheit, die laufend zu aufeinander folgenden Aufnahmezeitpunkten jeweils ein Höhenprofil der Person erstellt, wobei im Höhenprofil eine Anzahl von zumindest zwei Punkten im Raum festgelegt ist, die auf der Oberfläche der Person oder auf der Oberfläche eines auf oder neben der Person befindlichen Gegenstands liegen,
   sowie
- eine Verarbeitungseinheit, die
   a) für jeden der Aufnahmezeitpunkte das jeweilige Höhenprofil in einer Datenstruktur abgespeichert und zur Verfügung hält,
   b) eine Anzahl von zu aufeinander folgenden Aufnahmezeitpunkten, insbesondere innerhalb eines Zeitbereichs von 3 bis 20 Sekunden, aufgenommenen Höhenprofilen zu einem Segment zusammenfasst,
   c) einen Bereich, der den Bauch- oder Brustbereich der Person in Abhängigkeit vom jeweiligen Referenzpunkt oder Referenzbereich angibt als Betrachtungsbereich auswählt,
   d) für jedes Höhenprofil innerhalb des Segments jeweils gesondert den Mittelwert der Abstände der innerhalb des Betrachtungsbereichs befindlichen Punkte des Höhenprofils zu einem Referenzpunkt oder Referenzgegenstand ermittelt, und für das Segment ein Signal ermittelt wird, dem zum jeweiligen Aufnahmezeitpunkt des Höhenprofils der für dieses Höhenprofil ermittelte Mittelwert zugeordnet ist, und
   e) insbesondere anschließend basierend auf diesem Signal oder dessen Signalamplitude einen die Atemtiefe charakterisierender Wert ermittelt.

Durch diese vorteilhaften Maßnahmen wird eine berührungslose Detektion der Atmung einer Person ermöglicht, die die zu untersuchende Person im Schlaf nicht stört. Insbesondere ist es nicht erforderlich, Sensoren am Körper der zu untersuchenden Person zu befestigen.

Eine vorteilhafte und numerisch einfache Ausführungsform der Erfindung sieht eine . Vorrichtung zur Bestimmung des zeitlichen Verlaufs der Atemtiefe einer, insbesondere schlafenden, Person vor, umfassend
- eine auf eine Schlafgelegenheit, insbesondere ein Bett, ausrichtbare Detektoreinheit, die laufend zu aufeinander folgenden Aufnahmezeitpunkten jeweils ein Höhenprofil der Person erstellt, wobei das Höhenprofil eine Anzahl von zumindest zwei Abstandswerten zur Festlegung jeweils eines Punktes im Raum aufweist, wobei die einzelnen Abstandswerte jeweils den Abstand des Schnittpunktes eines relativ zur Detektoreinheit vorab festgelegten, insbesondere von der Detektoreinheit ausgehenden, Strahls mit der Oberfläche der Person oder der Oberfläche eines auf der oder neben der Person befindlichen Gegenstands von einem Referenzpunkt oder einer Referenzebene angeben sowie
- eine Verarbeitungseinheit, die
   a) für jeden der Aufnahmezeitpunkte jeweils eine Datenstruktur erstellt, die das jeweilige Höhenprofil enthält, wobei sämtliche so erstellte Datenstrukturen jeweils dieselbe Größe aufweisen und jeweils Speicherpositionen für die einzelnen Abstandswerte des Höhenprofils aufweisen,
   b) eine Anzahl von zu aufeinander folgenden Aufnahmezeitpunkten, insbesondere innerhalb eines Zeitbereichs von 3 bis 20 Sekunden, aufgenommenen Höhenprofilen zu einem Segment zusammenfasst,
   c) eine Anzahl von Speicherpositionen der Datenstruktur, in denen Abstandswerte abgespeichert sind, die den Abstand des Bauch- oder Brustbereichs der Person in Abhängigkeit vom jeweiligen Referenzpunkt oder Referenzbereich angeben, als Betrachtungsbereich auswählt,
   d) für jedes Höhenprofil innerhalb eines Segments jeweils gesondert den Mittelwert der innerhalb des Betrachtungsbereichs befindlichen Abstandswerte ermittelt, und für das Segment ein Signal ermittelt, dem zum jeweiligen Aufnahmezeitpunkt des Höhenprofils der für dieses Höhenprofil ermittelte Mittelwert zugeordnet ist,
   e) insbesondere anschließend basierend auf diesem Signal oder dessen Signalamplitude einen die Atemtiefe charakterisierenden Wert ermittelt.

Eine alternative vorteilhafte und numerisch präzise Ausführungsform der Erfindung, die die Verwendung von Höhenprofilen in Form von allgemeinen Punktwolken ermöglicht, sieht vor, dass die Detektoreinheit die Höhenprofile in Form von Punktwolken mit einer Anzahl von zumindest zwei Punkten im Raum erstellt, wobei die Punkte auf der Oberfläche der Person oder auf der Oberfläche eines auf oder neben der Person befindlichen Gegenstands liegen.

Bei einer Vorrichtung zur Bestimmung zeitlichen Verlaufs der Atemtiefe einer, insbesondere schlafenden, Person, sieht die Erfindung vor, dass die Verarbeitungseinheit zur Charakterisierung der Atemtiefe in einem Segment aus dem Signal zumindest ein Maximum und zumindest ein Minimum extrahiert und die zumindest eine Differenz zwischen dem Maximum und dem Minimum als die Atemtiefe charakterisierenden Wert für den dem Segment zugeordneten Zeitbereich zur Verfügung hält und gegebenenfalls heranzieht.

Eine numerisch einfache Filterung des Signals, die störendes Rauschen weitestgehend unterdrückt sieht vor, die Verarbeitungseinheit das Signal einer spektralen Transformation, insbesondere Fourier-Transformation, Cosinustransformation oder Wavelet-Transformation, unterzieht und innerhalb eines vorgegebenen Frequenzbands, insbesondere von 0,1 Hz bis 1 Hz, nach demjenigen Spektralanteil mit der höchsten Signalenergie sucht und die Signalenergie dieses Spektralanteils zur Charakterisierung der Atemtiefe in diesem Segment heranzieht.

Zur rauscharmen Extraktion von Atembewegungen, aus denen letztlich eine besonders gute Extraktion der Atemtiefe möglich ist, kann vorgesehen sein, dass die Verarbeitungseinheit das einem Segment zugeordnete Signal nach seiner Erstellung, insbesondere vor der Bestimmung des die Atemtiefe charakterisierenden Werts, einer Rauschfilterung unterzieht, wobei sie insbesondere
a) Signalanteile mit einer Frequenz von mehr als 0,5 Hz unterdrückt, und/oder
b) Gleichanteile unterdrückt.

Der Verlauf der Atemtiefe über die Zeit kann vorteilhaft ermittelt werden, indem die Verarbeitungseinheit für eine Anzahl von überlappenden oder nicht überlappenden Segmenten jeweils separat die Atemtiefe ermittelt.

Eine vorteilhafte adaptive Anpassung des Betrachtungsbereichs, die regelmäßig mit geringem Aufwand vorgenommen werden kann, sieht vor, die Verarbeitungseinheit den Betrachtungsbereich für jedes Segment separat, insbesondere auf Grundlage des für das jeweils vorangehende Segment ermittelten Betrachtungsbereichs ermittelt.

Besonders aussagekräftige und numerisch einfach handhabbare Höhenprofile können erstellt werden, indem die Verarbeitungseinheit
- das Höhenprofil durch eine zweidimensionale Matrixdatenstruktur umfassend eine Anzahl von Zeilen und Spalten charakterisiert,
- eine Anzahl von in Zeilen und Spalten rasterförmig angeordneten Positionen vorgibt, an denen jeweils die Abstandswerte des Höhenprofils bestimmt
- die Matrixdatenstruktur ein Raster von derselben Größe und Struktur aufweist, und
- die Matrixdatenstruktur erstellt, indem sie die an den jeweiligen Positionen aufgenommenen Abstandswerte an den den Positionen im Raster entsprechenden Speicherpositionen in der Matrixdatenstruktur abspeichert und zur Verfügung hält.

Um eine raschere Verarbeitung der Daten zu ermöglichen, den erforderlichen Speicherplatz zu reduzieren sowie das auftretende Rauschen zu reduzieren, kann vorgesehen sein, dass die Verarbeitungseinheit die Matrixdatenstruktur nach ihrer Erstellung in ihren Abmessungen durch eine reduzierte Matrixdatenstruktur ersetzt, wobei sie für rechteckige, insbesondere die gesamte Matrixdatenstruktur abdeckende, und nicht überlappende Bildbereiche von jeweils gleicher Größe in der Matrixdatenstruktur jeweils einen mittleren Abstandswert ermittelt und diesen mittleren Abstandswert dem bezüglich seiner Lage entsprechenden Bildpunkt der reduzierten Matrixdatenstruktur zuordnet.

Eine vorteilhafte Methode zur Bestimmung des Betrachtungsbereichs sieht vor, dass die Verarbeitungseinheit den Betrachtungsbereich festlegt, indem sie
a) in der im Höhenprofil, insbesondere in der Matrixdatenstruktur oder in der Punktwolke, eine Anzahl von möglichen Betrachtungsbereichen, vorab vorgibt,
b) für das jeweilige Segment die jeweilige Atemtiefe unter Zugrundelegung jedes der möglichen Betrachtungsbereiche ermittelt, und
c) denjenigen vorgegebenen möglichen Betrachtungsbereich als Betrachtungsbereich auswählt und heranzieht, für den die ermittelte Atemtiefe am größten ist.

Eine zweite vorteilhafte Methode zur Bestimmung des Betrachtungsbereichs, die besonders auf anatomische Gegebenheiten Rücksicht nimmt, sieht vor, dass die Verarbeitungseinheit den Betrachtungsbereich festlegt, indem sie
a) im Höhenprofil, insbesondere in der Matrixdatenstruktur oder in der Punktwolke, mittels Objekterkennung nach Bereichen sucht oder Mittel zur Auswahl von Bereichen zur Verfügung stellt, die einem menschlichen Kopf und Torso entsprechen, und
b) den den Torso abbildenden Bereich der des Höhenprofils, insbesondere der Matrixdatenstruktur oder der Punktwolke, oder ein Teilbereich dieses Bereichs als Betrachtungsbereich auswählt, wobei sie insbesondere
   - zur Detektion des Verlaufs oder der Atemtiefe der Brustatmung den dem Kopf nahen Teilbereich dieses Bereichs als Betrachtungsbereich auswählt und/oder
   - zur Detektion des Verlaufs oder der Atemtiefe der Bauchatmung den dem Kopf fernen Teilbereich dieses Bereichs als Betrachtungsbereich auswählt.

Eine rasche Anpassung des Betrachtungsbereichs kann erreicht werden, indem die Verarbeitungseinheit für jedes zu untersuchende Segment oder für einzelne Höhenprofile eines Segments den Betrachtungsbereich mittels Objekterkennung an die im Segment vorliegenden Höhenprofile ausgehend von der Position des ausgewählten Betrachtungsbereichs im jeweils vorangehenden Segment adaptiv neu ermittelt.

Eine dritte vorteilhafte Methode zur Bestimmung des Betrachtungsbereichs, die numerisch stabil und effizient ausführbar ist, sieht vor, dass die Verarbeitungseinheit den Betrachtungsbereich festlegt, indem sie
a) für sämtliche Punkte des Höhenprofils, insbesondere für alle Einträge der Matrixdatenstruktur, separat die Varianz der Werte über das jeweilige Segment bestimmt, und
b) einen Bereich oder mehrere Bereiche mit jeweils zusammenhängenden Punkten des Höhenprofils, deren jeweilige Varianz oberhalb eines unteren Schwellenwerts oder innerhalb eines vorgegebenen Intervalls liegt, als Betrachtungsbereich auswählt, insbesondere
   - indem sie zusammenhängende Bereiche als Betrachtungsbereich auswählt, deren Größe eine vorgegebene Anzahl von Punkten übersteigt
   - wobei insbesondere ein Bereich dann als zusammenhängend gilt, wenn jeder Punkt des Bereichs ausgehend von einem anderen Punkt innerhalb dieses Bereichs über jeweils benachbarte Pixel erreichbar ist,
   - wobei vorzugsweise Punkte des Höhenprofils dann als benachbart gilt,
      - wenn sie durch benachbarte Einträge der Matrix-Datenstruktur festgelegt sind oder
      - wenn sie oder ihre Projektion auf eine horizontale Ebene im Raum einen einen Schwellenwert unterschreitenden Abstand voneinander aufweisen.

Um Situationen, bei denen sich die zu untersuchende Person bewegt, detektieren zu können und die in diesen Situationen aufgenommenen fehlerhaften Messwerte verwerfen zu können, kann vorgesehen sein, die Verarbeitungseinheit den Betrachtungsbereich in zwei oder mehreren aufeinander folgenden Segmenten separat ermittelt und ein Höhenprofil verwirft und gegebenenfalls eine Fehlermeldung ausgibt, wenn sich der Betrachtungsbereich, insbesondere dessen Größe und/oder dessen Schwerpunkt, gegenüber dem jeweils vorangehenden Segment um einen vorgegebenen Schwellenwert verschiebt.

Zur verbesserten Detektion des Aussetzens der Atmung ist zusätzlich eine akustische Kontrolle vorgesehen. Hierbei ist vorgesehen, dass der Verarbeitungseinheit ein Mikrofon vorgeschaltet ist, das gleichzeitig parallel zur Aufnahme des Abstands der Person den von der Person abgegebene Schall in Form eines Audio-Signals an seinem Ausgang zur Verfügung hält und dieses Audio-Signal der Verarbeitungseinheit zugeführt ist, und dass die Verarbeitungseinheit
a) das Audio-Signal in eine Anzahl von Audio-Segmente, insbesondere mit einer Länge von 100 ms bis 1 Sekunde, unterteilt und für jedes der Audio-Segmente untersucht, ob in diesem menschliche Atemgeräusche oder andere Geräusche zu hören sind, und für jedes Audio-Segment jeweils ein Klassifikationsergebnis zur Verfügung hält,
c) zeitgleich aufgenommene Audio-Segmente und Segmente einander zuordnet, und
d) nach Audio-Segmenten oder Segmenten mit geringer Atemtiefe oder mit fehlenden Atemgeräuschen sucht und die einem solchen Audio-Segment oder Segment zugeordneten Segmente oder Audio-Segmente ebenfalls auf das Vorliegen geringer Atemtiefe oder fehlender Atemgeräuschen untersucht, und
e) für den Fall, dass in einander zugeordneten Segmenten und Audio-Segmenten jeweils geringe Atemtiefe und fehlende Atemgeräusche detektiert wurden, das Fehlen der Atmung der Person feststellt.

Eine besonders bevorzugte Ausführungsform der Erfindung, die eine Detektion des Beginns und des Endes von Atemaussetzern ermöglicht, sieht vor, dass dass die Verarbeitungseinheit
a) ein Klassifikationssignal erstellt, das für jedes Audio-Segment jeweils zumindest ein Klassifikationsergebnis enthält, das die Art und Stärke des jeweiligen Geräuschs im Zeitbereich des jeweiligen Audio-Segment angibt,
b) jeweils eine Anzahl von aufeinander folgenden Audio-Segmente zu weiteren Audio-Segmenten, insbesondere mit einer Dauer von 5 bis 10 Sekunden zusammenfasst, die vorzugsweise dieselben Zeitbereiche umfassen wie die Segmente,
c) über die innerhalb eines weiteren Audio-Segments enthaltenen Klassifikationsergebnisse eine Mittelung vornimmt und dem weiteren Audio-Segment den jeweiligen Mittelwert zuweist,
d) ein weiteres Klassifikationssignal durch Interpolation der Mittelwerte der weiteren Audio-Segmente erstellt,
e) im weiteren Klassifikationssignal sowie im Atemtiefesignal nach Zeitpunkten sucht, zu denen in den beiden Signalen starke Änderungen auftreten, und
   die so erkannten Zeitpunkte als umso relevanter eingestuft, je stärker die Änderung des jeweiligen Signals zum jeweiligen Zeitpunkt ist, wobei sie diesen Zeitpunkten jeweils ein diesbezügliches Relevanzmaß zuordnet, und
f) jene Zeitpunkten als Startpunkte oder Endpunkte eines Atemstillstandes detektiert, für welche der Betrag des Relevanzwerts über dem Betrag eines Schwellwert liegt, wobei sie insbesondere
   - der Schwellenwert bildet, indem sie über einen Zeitbereich, insbesondere vor und/oder nach dem Vergleichszeitpunkt mit dem Referenzmaß ein Mittelwert des Referenzmaßes bildet und der Schwellenwert im Bereich zwischen 120% und 200% dieses Mittelwerts festlegt.

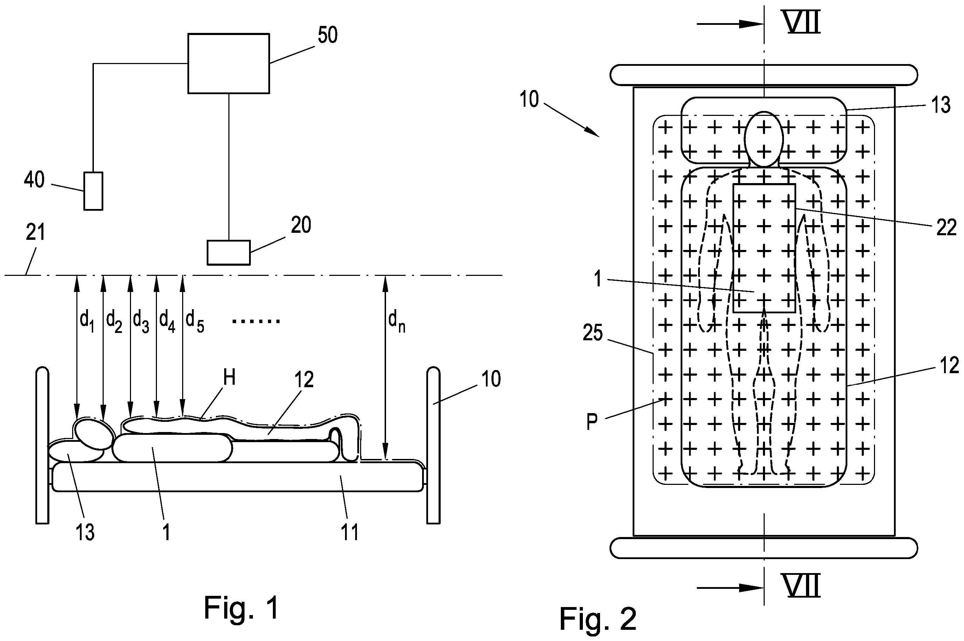

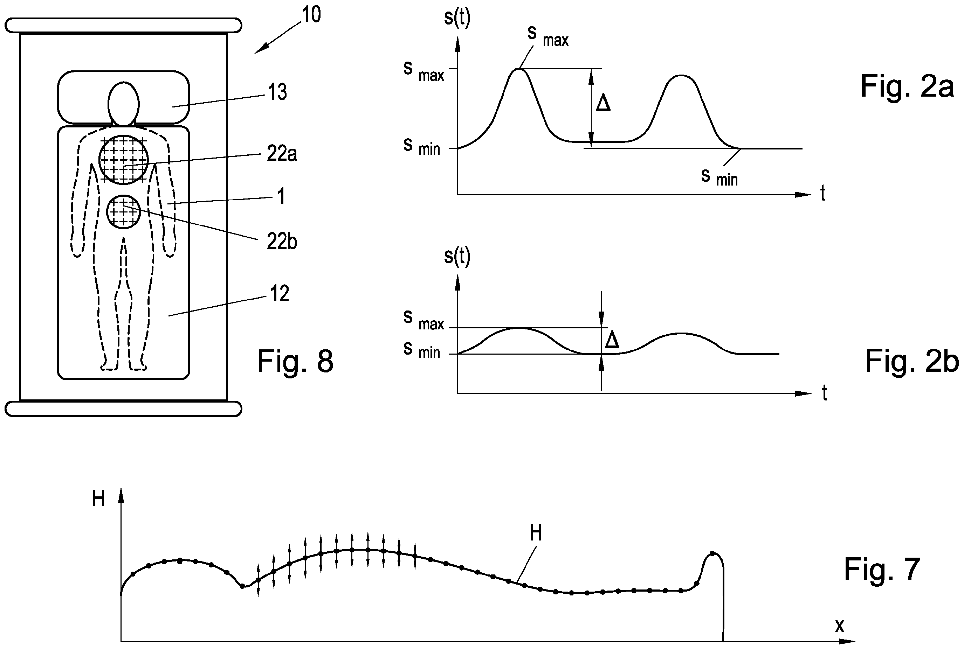

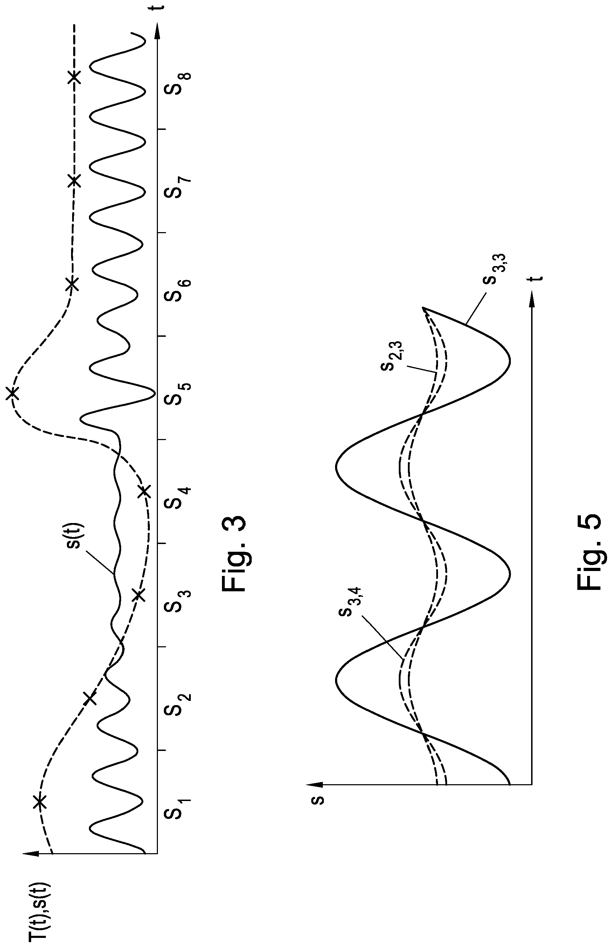

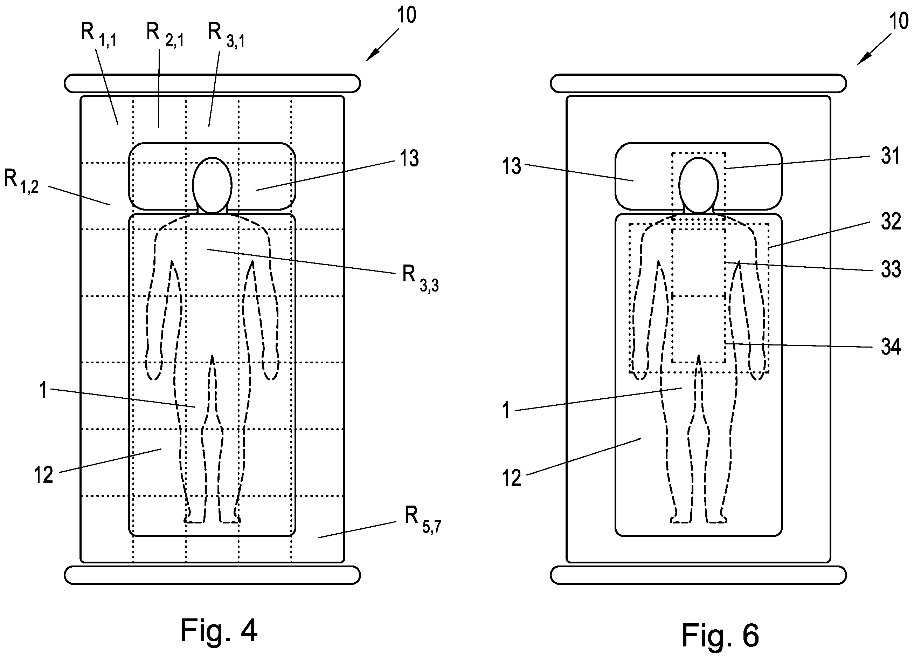

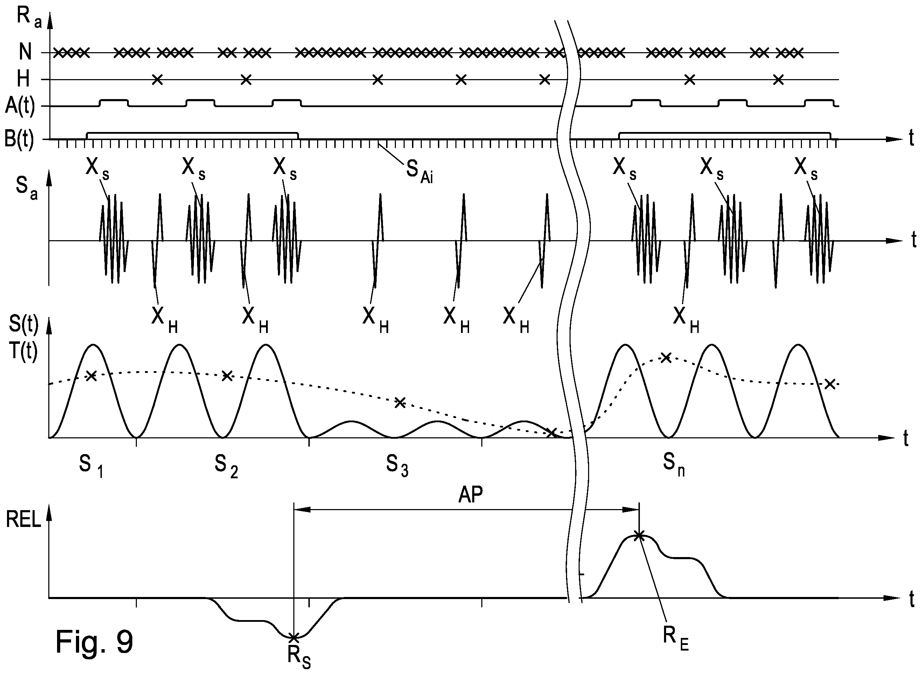

Mehrere Ausführungsbeispiele und Varianten der Erfindung werden anhand der folgenden Zeichnungsfiguren näher beschrieben. **Fig. 1** zeigt ein Bett mit einer schlafenden Person. **Fig. 2** zeigt eine Ansicht des in **Fig. 1** dargestellten Betts von oben. **Fig. 2a** zeigt einen Signalverlauf des Signals bei einer Person mit normaler Atmung. **Fig. 2b** zeigt einen Signalverlauf des Signals bei einer Person mit einer Obstruktion der Atmung. In **Fig. 3** ist eine Folge von Signalen von zeitlich aufeinander folgenden Segmenten dargestellt. **Fig. 4** und **Fig. 5** zeigen eine erste Vorgehensweise zur Bestimmung des Betrachtungsbereichs. **Fig. 6** zeigt ein alternatives Vorgehen zur Bestimmung des Betrachtungsbereichs unter Zuhilfenahme von Objekterkennungsalgorithmen. **Fig. 7** und **Fig. 8** zeigen eine dritte Vorgehensweise zur Bestimmung des Betrachtungsbereichs. **Fig. 9** zeigt eine Kombination eines auf der Auswertung von Höhenprofilen beruhenden Verfahrens mit einem akustischen Verfahren zur Erkennung von Atemaussetzern.

In **Fig. 1** ist ein Bett 10 von der Seite dargestellt. Das Bett 10 verfügt über einen Korpus, eine Matratze 11, ein Kissen 13 und eine Bettdecke 12. Im Bett 10 liegt eine schlafende Person 1, deren Kopf auf dem Kissen 13 liegt und dessen Oberkörper und Beine von der Bettdecke 12 abgedeckt sind.

Oberhalb der Person ist eine Detektoreinheit 20 angeordnet, mit der der Abstand der Person 1 bzw. von einer Vielzahl von Punkten auf der Person von einer vorgegebenen Position, die relativ zur Detektoreinheit 20 festgelegt ist, ermittelt werden kann. Der Detektoreinheit 20 ist eine Verarbeitungseinheit 50 nachgeschaltet, die die im Folgenden dargestellten numerischen Verarbeitungsschritte durchführt.

Im vorliegenden Fall handelt es sich bei der Detektoreinheit 20 um eine Einheit, die jeweils den Normalabstand eines Punkts auf der Person 1 von einer oberhalb der Person 1 horizontal verlaufenden Referenzebene 21 angibt. Die Detektoreinheit 20 misst den jeweiligen Abstand d₁, ..., dₙ an n unterschiedlichen Positionen, die im vorliegenden Ausführungsbeispiel rasterförmig in Form von Zeilen und Spalten angeordnet sind, und erstellt aufgrund dieser Abstandsmesswerte ein Höhenprofil H.

Im vorliegenden Ausführungsbeispiel wird zur Erstellung eines Höhenprofils H eine Detektoreinheit 20 verwendet, die etwa 2 Meter über der Person angeordnet ist. Eine solche Detektoreinheit kann auf unterschiedliche Weise aufgebaut sein.

In einer ersten Ausführungsvariante kann die Detektoreinheit als Time-Of-Flight Kamera (TOF-Kamera) ausgebildet sein. Diese bestimmt den Abstand zu einem Objekt wird mithilfe der "Flugzeit" eines ausgesendeten Lichtimpulses. Dabei entstehen Abstandsmessungen mit einer lateralen Auflösung, die sich typischerweise ca. im Bereich von 320x240 Pixel bewegt. Die konkrete Funktionsweise einer solchen TOF-Kamera ist aus dem Stand der Technik bekannt und ist in Hansard, M., Lee, S., Choi, O., and Horaud, R. (2013). Time-of-fight cameras. Springer näher dargestellt.

Alternativ kann ein Höhenprofil auch mittels Lichtschnittverfahren bestimmt werden. Bei diesen wird mithilfe von einer Lichtquelle, die auf die Person 1 gerichtet ist die Oberfläche trianguliert. Man erhält keine absoluten Abstandsmessungen, sondern lediglich ein Höhenprofil. Da im Rahmen der Erfindung ohnedies keine absoluten Messwerte sondern lediglich die relative Werte wie Varianz bzw. die Änderung der Varianz über die Zeit verwendet werden, können auch Höhenprofile ohne weiteres für die Erfindung verwendet werden. Die folgende Veröffentlichung beschreibt ein System, das Höhenprofile mit einer Framerate von ca. 40 fps und bei einer Auflösung von 640x480 Pixeln liefert: Oike, Y., Shintaku, H., Takayama, S., Ikeda, M., and Asada, K. (2003). Real-time and highresolution 3d imaging system using light-section method and smart cmos sensor. In Sensors, 2003. Proceedings of IEEE, volume 1, pages 502-507. IEEE.

Eine weitere alternative Methode zur Aufnahme eines Höhenprofils bedient sich einer Radarmessung. Hierfür werden Radarmesseinrichtungen, gegebenenfalls in ihrer Richtung steuerbare Radarmesseinrichtungen, sogenannte Phased Arrays verwendet. Mithilfe von Phasenverschiebungen in dem Radarimpuls im Antennenarray kann der Impuls auf einen gewissen Punkt auf dem Körper der Person 1 gelenkt werden und damit der Raum abgetastet werden. Grundsätzlich kann mit einer solchen Radarmesseinrichtung eine hohe räumliche Auflösung erzielt werden. Es ist auch ohne weiteres möglich, 30 Höhenprofile pro Sekunde zu erzielen. Der konkrete Aufbau solcher Radarmesseinrichtungen ist in Mailloux, R. J. (2005). Phased array antenna handbook. Artech House Boston beschrieben.

**Fig. 2** zeigt eine Ansicht des in **Fig. 1** dargestellten Betts 10 von oben, wobei an einer Anzahl n von Positionen jeweils der Abstand d₁, ..., dₙ der Person 1 und/oder der die Person 1 überdeckenden Bettdecke 12 und/oder des Kissens 13 von der Referenzebene 21 gemessen wird. Die einzelnen Positionen an denen Messungen vorgenommen werden, sind rasterförmig angeordnet und in **Fig. 2** durch Kreuze dargestellt. Diese Positionen lassen sich durch die Angabe einer x-Koordinate und einer y-Koordinate eindeutig beschreiben. Aus den einzelnen ermittelten Abständen d₁, ..., dₙ wird laufend zu aufeinander folgenden Aufnahmezeitpunkten t₁, ..., tₚ ein Höhenprofil H der Person 1 erstellt. Im vorliegenden Fall enthält das Höhenprofil die einzelnen ermittelten Abstände d₁, ..., dₙ und wird in Form einer Matrixdatenstruktur zur Verfügung gehalten.

Eine solche Darstellung des Höhenprofils ist jedoch für die Erfindung aus mehreren Gründen nicht zwingend.

Das Höhenprofil muss nicht notwendigerweise an Punkten den Abstand der Oberfläche der Person oder der Oberfläche eines auf der oder neben der Person befindlichen Gegenstands zu einer Referenzebene 21 geben, die lateral rasterförmig angeordnet sind. Vielmehr ist es auch denkbar, dass die Abstände entlang von vorgegebenen Strahlen, die von der Detektoreinheit ausgehen gemessen werden und dann die vertikalen Abstände von dem jeweiligen Schnittpunkt mit der Oberfläche zur Referenzebene mithilfe des gemessenen Abstandes und des jeweilig verwendeten Messwinkels errechnet werden. Die Winkel der verschiedenen Messstrahlen können dabei so gewählt werden, dass sich bei Auftreffen auf eine Ebene, die parallel zur Referenzebene 21 ist eine rasterförmige Anordnung erben würde. Selbst bei dieser sehr speziellen Auswahl von Messstrahlen ergeben sich laterale Messpunkte, deren x- und y-Koordinaten von der regelmäßigen Rasteranordnung in **Fig. 2** abweichen, sobald die gemessene Oberfläche von einer Ebene abweicht. Da jedoch die Schwankung der Höhe der gemessenen Oberfläche eines auf der oder neben der Person befindlichen Gegenstands im Verhältnis zum Abstand der Detektoreinheit typischerweise klein ist können die lateralen Koordinaten bei obig genannter Anordnung der Messstrahlen annähernd in der Anordnung von **Fig. 2** und damit in einer Matrixdatenstruktur angenommen werden.

Im Allgemeinen geben die einzelnen Abstandswerte jeweils den Abstand des Schnittpunktes eines relativ zur Detektoreinheit vorab festgelegten, insbesondere von der Detektoreinheit ausgehenden, Strahls mit der Oberfläche der Person (1) oder der Oberfläche eines auf der oder neben der Person (1) befindlichen Gegenstands von einem Referenzpunkt oder einer Referenzebene (21) an.

Weiters ist es nicht notwendig, dass die einzelnen zur Verfügung stehenden Abstandswerte in einer Matrixdatenstruktur angeordnet sind. Es ist auch möglich, dass eine Datenstruktur mit abweichendem Aufbau gewählt wird und nur Abstände entlang ganz bestimmter Strahlen ermittelt werden.

Im Rahmen der Erfindung ist es aber auch möglich, das Höhenprofil in Form einer Punktwolke anzugeben. Die Punktwolke umfasst eine Liste von Punkten jeweils unter Angabe ihrer jeweiligen Koordinaten in drei Dimensionen bezogen auf einen Bezugspunkt im Raum.

Auch andere Darstellungen der Relativposition der Oberfläche der Person 1 oder der Oberfläche eines auf der oder neben der Person 1 befindlichen Gegenstands in Bezug der Detektoreinheit oder zum Raum sind möglich, wenn einzelne Punkte auf dieser Oberfläche bestimmbar sind.

Der von der Detektoreinheit 20 aufgenommene bzw. der von den Positionen abgedeckte Bereich 25 beschränkt sich auf den Körper der Person 1, um möglichst wenige irrelevante Bewegungen der Person 1 oder Bewegungsartefakte zu detektieren.

Die Abstandsmesswerte d₁, ..., dₙ des Höhenprofils H werden im Folgenden mit H(x,y,t) bezeichnet, wobei die ersten beiden Koordinaten die räumliche Position bezeichnen und die letzte Koordinate t den Aufnahmezeitpunkt t₁, ..., tₚ bezeichnet. Es wird angenommen, dass *x* ∈ [0, *...,.X*] und *y* ∈ [0, ...,*Y*], das heißt die räumliche Auflösung des Datenstromes ist *X* × *Y*. Die dritte Koordinate t steht für die Zeit und bezeichnet den Aufnahmezeitpunkt t₁, ..., tₚ, der in Vielfachen der zeitlichen Samplingdichte bzw. Abtastrate, z.B. 1/30s für 30 Frames/Sekunde, angegeben wird.

Insgesamt wird im Zuge der Aufnahme eine dreidimensionale Datenstruktur erstellt, bei der für jeden Aufnahmezeitpunkt t₁, ..., tₚ jeweils eine zweidimensionale Matrixdatenstruktur zur Verfügung steht, deren Einträge jeweils dem Abstand der Person 1 von der Referenzebene 21 in einem durch die Position der Einträge in der Matrixdatenstruktur definierten Bereich entsprechen. Jede so erstellte Matrixdatenstruktur ist gleich groß. Alle Matrixdatenstrukturen enthalten jeweils Speicherpositionen für die einzelnen Abstandsmesswerte d₁, ..., dₙ oder für daraus abgeleitete Werte.

In einem optionalen Schritt kann die räumliche Auflösung der Matrixdatenstruktur verringert werden, indem mehrere Einträge der Matrixdatenstruktur zu einem Eintrag einer reduzierten Matrixdatenstruktur zusammengefasst werden. Dies kann im einfachsten Fall bedeuten, dass nur einzelne der Abstandsmesswerte d für die Bildung der reduzierten Matrixdatenstruktur herangezogen werden und die übrigen Abstandsmesswerte verworfen werden. Für ganzzahlige Parameter a und b bekommt man eine reduzierte Matrixdatenstruktur Hᵣ(x,y,t)=H(ax,by,t), deren Speicherbedarf um das (a x b)-fache kleiner ist als der Speicherbedarf der Matrixdatenstruktur.

Um ein besseres und robusteres Ergebnis zu bekommen, kann vor der Reduktion der Auflösung die Matrixdatenstruktur geglättet oder gefiltert werden. Dazu berechnet man die zweidimensionale Fouriertransformation von H bezüglich der ersten beiden Koordinaten, multipliziert mit einer Filtertransferfunktion, periodisiert dieses Signal mit den Parametern X/a und Y/b und berechnet dann die inverse Fouriertransformation, um die reduzierte Matrixdatenstruktur zu erhalten. Das vorstehend beschriebene Subsampling ist lediglich ein Spezialfall davon mit einer konstanten Filtertransferfunktion. Auch eine Mittelwertbildung über eine Anzahl von innerhalb von rechteckigen Bereichen der Matrixdatenstruktur der Größe a x b kann mithilfe einer Filterung mit anschließendem Subsampling realisiert werden.

In einem weiteren Schritt wird das Aufnahmeintervall, in dem Höhenprofile H erstellt wurden, in vorzugsweise aneinander anschließende und nicht überlappende Zeitabschnitte von 5 bis 10 Sekunden unterteilt. Es werden Segmente S₁, ..., Sₘ erstellt, die die einzelnen ermittelten Matrixdatenstrukturen, die innerhalb eines Zeitabschnitts aufgenommen wurden, enthalten. Alternativ ist es auch möglich, dass die einzelnen Zeitabschnitte überlappen oder dass Höhenprofile einzelner Zeitbereiche in keinem der Segmente S₁, ..., Sₙ enthalten sind.

Für jedes der Segmente Sᵢ ergibt sich eine Datenstruktur Hᵢ(x,y,t), wobei *x* ∈ [0, ....,*X*] ,*y* ∈ [0, ...,*Y*] und *t* ∈ *S*ᵢ. Darüber hinaus werden die Zeitpunkte, zu denen ein Segment beginnt mit T_{3D,i} bezeichnet. Im Folgenden wird beschrieben wie für jeden einzelnen dieser Zeitblöcke Atemaktivität festgestellt werden kann und optional auch die Atemtiefe gemessen werden kann.

Für jedes Segment Sᵢ wird ein Betrachtungsbereich 22 ausgewählt, der diejenige Region oder Regionen der Matrixdatenstruktur bezeichnet, in der oder denen eine Atembewegung erwartet wird. Diese kann von Hand voreingestellt werden und über die einzelnen Segmente Sᵢ hinweg konstant gewählt werden.

Es besteht jedoch auch die Möglichkeit, den Betrachtungsbereich 22 an Bewegungen der Person anzupassen. Da die einzelnen Segmente Sᵢ jedoch nur Matrixdatenstrukturen aus Zeitbereichen von etwa 10 Sekunden enthalten, ist eine Anpassung innerhalb eines Segments Sᵢ in den meisten Fällen nicht erforderlich. Innerhalb eines Segments Sᵢ wird der Betrachtungsbereich 22 üblicherweise konstant angenommen.

Die Anpassung des Betrachtungsbereichs 22 für die einzelnen Segmente Sᵢ hat den Vorteil, dass eine einfache Detektion der Atmung der Person 1 auch möglich ist, wenn diese sich im Schlaf bewegt. Vorteilhaft wird der Betrachtungsbereich 22 automatisch bestimmt. Weiter unten werden drei verschiedene Techniken beschrieben, um den Betrachtungsbereich 22 für jedes Segment Sᵢ automatisch zu adaptieren, um möglichen Bewegungen der Person 1 folgen zu können.

Nachdem der Betrachtungsbereich 22 festgelegt ist, wird ein zeitliches Signal sᵢ erstellt, bei dem für jede Matrixdatenstruktur jeweils ein Signalwert sᵢ(t) ermittelt wird. Der Signalwert wird im vorliegenden Fall ermittelt, indem für den jeweiligen Zeitpunkt t der Mittelwert der Abstandswerte H(x, y, t) ermittelt wird, deren Position innerhalb des Betrachtungsbereichs 22 liegt. Man erhält auf diese Weise ein Signal sᵢ(t), das nur mehr von der Zeit abhängig ist.

Dieses Signal s(t) kann eventuell Rauschen enthalten. Um aus diesem verrauschten Signal s(t) die Atmung, sowie optional auch ein Maß für die Atemtiefe zu erhalten kann dieses Signal s(t) einer Rauschfilterung unterzogen werden. Eine vorteilhafte Rauschunterdrückung kann beispielsweise erreicht werden, indem Signalanteile mit einer Frequenz von mehr als 1 Hz unterdrückt werden. Alternativ oder zusätzlich kann auch vorgesehen sein, Gleichanteile bzw. Frequenzen bis zu 0.1 Hz zu unterdrücken. Mit dieser Filterung kann erreicht werden, dass nur Frequenzen, die für die Feststellung der Atmung relevant sind übrig bleiben. Das so erhaltene Signal sᵢ(t) stimmt mit den tatsächlich durchgeführten Atembewegungen gut überein.

Die Atemtiefe T kann besonders vorteilhaft von diesem Signal s(t) abgeleitet werden. Hierfür gibt es eine Vielzahl an Möglichkeiten, von denen zwei näher dargestellt werden. **Fig. 2a** zeigt einen ersten Signalverlauf des Signals s(t), wobei innerhalb des Signalverlaufs nach einem Minimum sₘᵢₙ und nach einem Maximum sₘₐₓ gesucht wird. Anschließend wird die Differenz Δ zwischen dem Minimum sₘᵢₙ und einem Maximum sₘₐₓ gebildet und diese Differenz Δ als Maß für die Atemtiefe T herangezogen.

Wie aus **Fig. 2b** ersichtlich, ist die Atmung der Person 1 wesentlich schwächer als bei dem in **Fig. 2a** dargestellten Signal. Aus diesem Grund ist auch die Differenz Δ zwischen dem Minimum sₘᵢₙ und einem Maximum sₘₐₓ kleiner als bei dem in **Fig. 2a** dargestellten Signal.

Sofern innerhalb eines Signals mehrere lokale Maxima und Minima aufgefunden werden, ist es auch möglich, als Atemtiefe T die größte Differenz zwischen je einem Minimum und dem jeweils nächstfolgenden lokalen Maximum heranzuziehen.

Alternativ ist es auch möglich, dass das Signal einer spektralen Transformation, insbesondere Fourier-Transformation, Cosinustransformation oder Wavelet-Transformation, unterworfen wird. Innerhalb eines vorgegebenen Frequenzbands, insbesondere von 0,1 Hz bis 1 Hz, wird nach demjenigen Spektralanteil mit der höchsten Signalenergie gesucht. Die Signalenergie dieses Spektralanteils wird zur Charakterisierung der Atemtiefe T in diesem Segment herangezogen.

In **Fig. 3** ist eine Folge von Signalen sᵢ(t) von zeitlich aufeinander folgenden Segmenten Sᵢ dargestellt, wobei während der Segmente S₃ und S₄ ein Atemaussetzer der Person 1 vorliegt. Weiters ist in **Fig. 3** jeweils auch über derselben Zeitachse die Atemtiefe Aᵢ für das jeweilige Segment Sᵢ dargestellt sowie eine Interpolationskurve T(t), die die Atemtiefe T für sämtliche Zeitpunkte innerhalb der dargestellten Segmente S₁, ..., S₇ angibt. Klar ersichtlich ist, dass die Werte A₁, ..., A₇ für die Atemtiefe während des Atemaussetzers in den Segmenten S₃ und S₄ gegenüber den übrigen Segmenten deutlich verringert sind.

Im Folgenden werden einzelne mögliche Vorgehensweisen zur automatischen Erkennung des Betrachtungsbereichs 22 innerhalb eines Segments Sᵢ gezeigt.

Bei einer ersten Vorgehensweise (**Fig. 4**) werden einzelne mögliche Betrachtungsbereichen R_{1,1}, ... vorgegeben. Anschließend wird gemäß den vorstehend genannten Methoden jeweils die Atemtiefe T in sämtlichen möglichen Betrachtungsbereiche R_{1,1}, ... ermittelt. In **Fig. 5** sind einzelne Signale s_{3,4}, s_{2,3} und s_{3,3} gezeigt, die auf Grundlage der möglichen Betrachtungsbereiche R_{3,4}, R_{2,3} und R_{3,3} erstellt wurden. Da der mögliche Betrachtungsbereich R_{3,3} genau den Brustkorbbereich der Person 1 abbildet, sind auch die Signalamplituden in diesem möglichen Betrachtungsbereich R_{3,3} größer als in den beiden anderen Betrachtungsfenstern R_{3,4}, R_{2,3}, die den Kopf bzw. die Arme der Person 1 abbilden. Aus diesem Grund wird der mögliche Betrachtungsbereich R_{3,3} als Betrachtungsbereich 22 für das jeweilige Segment Sᵢ ausgewählt.

Die Position und Größe des Betrachtungsbereichs R_{3,3} kann noch verbessert werden, indem versucht wird, die Eckpunkte des den Betrachtungsbereich festlegenden Rechtecks zu verschieben und die Atemtiefe T unter Zugrundelegung dieses modifizierten Betrachtungsbereichs erneut zu berechnen. Durch Variation der Eckpunkte des Betrachtungsbereichs kann dieser adaptiv so lange verbessert werden, bis eine Erhöhung der ermittelten Atemtiefe durch Verschiebung der Eckpunkte nicht mehr erreicht werden kann.

Eine weitere, in **Fig. 6** dargestellte Vorgehensweise zur Bestimmung des Betrachtungsbereichs 22 kann mittels Objekterkennung vorgenommen werden. Zunächst wird in einer oder mehreren der Matrixdatenstrukturen des Segments Sᵢ mittels Objekterkennung nach Bereichen 31, 32 gesucht, die einem menschlichen Kopf und Oberkörper bzw. Torso entsprechen. Der den Torso abbildende Bereich 32 der Matrixdatenstruktur oder ein Teilbereich dieses Bereichs kann als Betrachtungsbereich 22 ausgewählt werden.

Zur Detektion der Brustatmung kann der dem Kopf 31 nahe Teilbereich 33 dieses Bereichs 32 als Betrachtungsbereich 22 ausgewählt werden. Zur Detektion der Bauchatmung kann der dem Kopf 31 ferne Teilbereich 34 dieses Bereichs 32 als Betrachtungsbereich 22 ausgewählt werden.

Für die Objekterkennung kommen mehrere verschiedene Bildverarbeitungsmethoden in Frage. Ziel der bekannten Objekterkennungsverfahren ist es, die Umrisse eines menschlichen Körpers automatisch oder halbautomatisch, d.h. zum Teil mit menschlicher Unterstützung, in einem 3D Bild oder Höhenprofil zu erkennen. Ein solches Vorgehen ist in den folgenden Veröffentlichungen beschrieben:
Gabriele Fanelli, Juergen Gall, and Luc Van Gool. Real time head pose estimation with random regression forests. In Computer Vision and Pattern Recognition (CVPR), pages 617-624, June 2011.

Jamie Shotton, Ross Girshick, Andrew Fitzgibbon, Toby Sharp, Mat Cook, Mark Finocchio, Richard Moore, Pushmeet Kohli, Antonio Criminisi, Alex Kipman, et al. Efficient human pose estimation from single depth images. Pattern Analysis and Machine Intelligence, IEEE Transactions on, 35(12):2821-2840, 2013.

Bei der Detektion von Körperstrukturen mittels Objekterkennung kommen statistische Lernmethoden zum Einsatz. Während die erste Veröffentlichung darauf abzielt die Haltung des Kopfes zu bestimmen, zielt die zweite Veröffentlichung darauf ab den Körper und im Speziellen die Gelenke zu lokalisieren. Mit beiden Verfahren kann auch die Position des Kopfes und des Torsos in dem Höhenprofil H bestimmt werden.

Eine weitere Möglichkeit besteht darin, das Höhenprofil H des Kopfes zunächst manuell festzulegen und dann in jedem Segment Sᵢ mithilfe einer Korrelation festzustellen, wo sich der Kopf zu dieser Zeit befindet. Auf die gleiche Weise kann auch der Torso in jedem Segment Sᵢ bzw. in jeder Matrixdatenstruktur innerhalb eines Segments Sᵢ gefunden werden. Ist die Lage des Torso und des Kopfes bekannt ist es einfach, den Torso in Brust und Bauchregion zu unterteilen. Dadurch kann man Atmung sowohl in der Brust, als auch in der Bauchgegend separat detektieren. Die gleiche Segmentierung des Körpers der Person kann mithilfe vieler verschiedener Algorithmen realisiert werden.

Kann in einem der Segmente Sᵢ ein Betrachtungsbereich 22 identifiziert werden, so kann in den meisten Fällen mit geringem Aufwand der Betrachtungsbereich 22 in dem Segment Sᵢ₊₁ identifiziert werden, das diesem Segment Sᵢ unmittelbar nachfolgt. Bei beiden vorab dargestellten Vorgehensweisen erleichtert die Kenntnis der ungefähren Position des Abbilds des Bauch- oder Brustbereichs das Auffinden des Bauch- oder Brustbereichs im jeweils nächsten Segment Sᵢ₊₁.

In **Fig. 7 und 8** ist eine dritte Möglichkeit zum Auffinden des Bauch- oder Brustbereichs der Person als Betrachtungsbereich 22 dargestellt. In einem ersten Schritt wird für sämtliche Einträge der Matrixdatenstruktur separat die Varianz der Werte über das jeweilige Segment Sᵢ bestimmt. Anschließend wird derjenige Bereich oder werden zusammenhängende Bereiche von Einträgen innerhalb der Matrixdatenstruktur als Betrachtungsbereich 22 ausgewählt, deren jeweilige Varianzen oberhalb eines unteren Schwellenwerts oder innerhalb eines vorgegebenen Intervalls liegen. **Fig. 7** zeigt einen Schnitt durch ein Höhenprofil einer Person entlang der Schnittlinie VII-VII aus **Fig. 2****.** Die strichlierte Linie stellt den zeitlichen Mittelwert der einzelnen Abstandswerte an der jeweiligen Position dar. Die Pfeile zeigen die Varianz an der jeweiligen Position. Es zeigt sich, dass im Bereich der Brust und im Bereich des Bauchs der Person größere Varianzen auftreten als im Rest des Körpers. Diese Bereiche werden als Betrachtungsbereiche 22a, 22b ausgewählt.

Um Fehldetektionen durch einzelne Artefakte vermeiden zu können, werden als Betrachtungsbereich ausschließlich zusammenhängende Bereiche 22a, 22b der Matrixdatenstruktur ausgewählt, deren Größe eine vorgegebene Anzahl von Einträgen übersteigt.

Diese Variante der Ermittlung des Betrachtungsbereichs 22 erlaubt auch die Detektion von Positionsänderungen der Person sowie von Messfehlern. Wird der Betrachtungsbereich 22 in zwei oder mehreren aufeinander folgenden Segmenten Sᵢ, ... separat ermittelt, so kann ein Höhenprofil verworfen werden und eine Fehlermeldung ausgegeben werden, wenn sich die Größe und der Schwerpunkt eines Betrachtungsbereichs 22a, 22b, gegenüber dem entsprechenden Betrachtungsbereich 22a, 22b im jeweils vorangehenden Segment Sᵢ um einen vorgegebenen Schwellenwert verschiebt oder ändert.

Es ist natürlich auch möglich, die vorstehend genannten Vorgehensweisen zur Bestimmung des Betrachtungsbereichs 22 zu kombinieren. Zum Beispiel kann mithilfe der Bildverarbeitungsmethoden zur Objekterkennung der Torso bestimmt werden und dann innerhalb dieses Bereichs nur diejenigen Einträge ausgewählt werden, für die eine signifikante Bewegung mithilfe der Varianz detektiert wurde. Alternativ ist es auch möglich, mehrere verschiedene mögliche Betrachtungsbereiche 22 auszuwählen und dann denjenigen Betrachtungsbereich 22, mit dem die größte Atemtiefe ermittelt wurde, zur Atemdetektion heranzuziehen.

In den dargestellten Ausführungsbeispielen wurde stets ein Höhenprofil verwendet, das in einer Matrixdatenstruktur abgespeichert ist. Die Erfindung erlaubt jedoch auch alternative Ausführungsformen, bei denen die Höhenprofile jeweils nur eine Anzahl von im Raum befindlichen Punkten festlegen. Es ist nicht erforderlich, dass die jeweiligen Punkte explizit durch Koordinaten festgelegt werden, solange der jeweilige Punkt aufgrund der Angaben im Höhenprofil H im Raum eindeutig festlegbar ist. Bei den vorliegenden Ausführungsbeispielen ist dies implizit durch die konkrete Position, an der der Abstand der Person 1 bestimmt wird, gegeben.

Alternativ ist es auch möglich, das Höhenprofil durch eine Punktwolke anzugeben, d.h. im Wesentlichen eine Liste von Punkten im Raum anzugeben. In einem solchen Fall kann der Betrachtungsbereich 22 als dreidimensionaler Bereich festgelegt werden, der sich im Brust- bzw. Bauchbereich der Person 1 befindet. Für die Bildung des jeweiligen Mittelwerts werden lediglich Punkte herangezogen, die sich innerhalb des jeweiligen Betrachtungsbereichs 22 befinden. Um sämtliche Hebungen und Senkungen des Brust- bzw. Bauchbereichs der Person 1 abzudecken, kann als Betrachtungsbereichs 22 ein Bereich gewählt werden, der auch ein über dem Brust- bzw. Bauchbereich liegendes Volumen bis etwa 10 cm über dem Brust- bzw. Bauchbereich umfasst.

Eine bevorzugte Ausführungsform der Erfindung bietet auch die Möglichkeit, die Atemtiefe und Atmung der Person 1 parallel zu den Höhenprofilen durch akustische Aufnahmen zu ermitteln. In **Fig. 1** ist ein optionales Mikrofon 40 dargestellt, mit dem ein Audio-Signal sₐ aufgenommen wird. Das vom Mikrofon 40 erstellte Audio-Signal sₐ wird digitalisiert und mit einer vorgegebenen Abtastrate abgespeichert und zur Verfügung gehalten. Das Mikrofon ist an die Verarbeitungseinheit 50 angeschlossen.

Das Audio-Signal sₐ wird zuerst in Audio-Segmente Sₐ mit einer vorgegebenen Länge von etwa 200ms unterteilt. Diese Audio-Segmente Sₐ können sich im Allgemeinen auch überlappen, im vorliegenden Fall werden jedoch aneinander anschließende, nicht überlappende Audio-Segmente Sₐ gewählt.

Optional können die Audio-Segmente Sₐ mit einer Fensterfunktion multipliziert werden, um bei den weiter unten beschriebenen Fourier-Methoden Randeffekte zu vermeiden. Darüber hinaus wird mit jedem Audio-Segment Sₐ auch ein Zeitpunkt T_{audio,i} verknüpft, der angibt, zu welchem Zeitpunkt das Segment fᵢ beginnt.

Jedes der Audio-Segmente S_{Ai} wird einer Klassifikation zugeführt, mit der erkannt wird, ob im jeweiligen Audio-Segment S_{Ai} kein Geräusch, ein Hintergrundgeräusch H, ein Atmungsgeräusch oder ein Schnarchgeräusch A/S zu hören ist. Für jedes Audio-Segment S_{Ai} wird jeweils ein Klassifikationsergebnis erstellt, das ja nachdem, ob kein Geräusch, ein Hintergrundgeräusch, ein Atmungsgeräusch oder ein Schnarchgeräusch zu hören ist, einen diesbezüglichen Wert N, H, A/S (**Fig. 9**) aufweist. Die Klassifikationsergebnisse werden zu einem diskreten zeitlichen Klassifikationssignal A(t) zusammengefasst.

Im Folgenden wird beschrieben, wie man mit statistischen Lernmethoden jedes dieser einzelnen Audio-Segmente S_{Ai} auf das Vorhandensein von Schnarchgeräuschen untersuchen kann.

In einem ersten Schritt kann zur Detektion von Schnarchgeräuschen in den einzelnen Audio-Segmenten S_{Ai} aus den Audio-Segmenten S_{Ai} ein Merkmalsvektor mᵢ extrahiert werden. Der Merkmalsvektor mᵢ für das i-te Segment Audio-Segment S_{Ai} wird direkt aus den Abtastwerten des jeweiligen Audio-Segments S_{Ai} berechnet. Der i-te Merkmalsvektor mᵢ kann von verschiedener Dimension sein, je nachdem welche Methoden zum Einsatz kommen, um die Merkmale zu berechnen. Im Folgenden werden einige verschiedene Techniken aufgezählt, die verwendet werden können, um Merkmale zu generieren.

Mithilfe der Fourier-Transformation eines Audio-Segments S_{Ai} kann eine Spektralanalyse des jeweiligen Audio-Segments S_{Ai} durchgeführt werden. Als Merkmale werden die Energien in gewissen Frequenzbändern verwendet, also die Summe der Betragsquadrate der Fourierkoeffizienten über gewisse vorspezifizierte Bänder. Dadurch erhält man für jedes Audio-Segment S_{Ai} einen Vektor mit einer Länge, die festgelegt ist durch die Anzahl der Bänder. Wird dieser Vektor noch einmal diskret Cosinus-transformiert, so erhält man ein weiteres mögliches Set an Koeffizienten des Merkmalsvektors mᵢ.

Ein weiteres mögliches Merkmal eines Merkmalsvektors mᵢ ist die Energie in einem Audio-Segment S_{Ai}. Darüber hinaus kann auch die Anzahl der Nulldurchgänge, also die Anzahl der Vorzeichenwechsel in einem Audio-Segment S_{Ai} als mögliches Merkmal eines Merkmalsvektors mᵢ herangezogen werden.

Nachdem festgelegt ist, wie sich die Merkmalsvektoren mᵢ zusammensetzen, wird eine statistische Lernmethode ausgewählt. Hierfür steht eine große Anzahl an möglichen Methoden im Stand der Technik zur Verfügung, daher wird hier nicht näher auf die verschiedenen Optionen eingegangen. Diese Methoden erlauben es, ein Audio-Segment S_{Ai} anhand eines Merkmalsvektors mᵢ als Schnarchgeräusch zu identifizieren. Dazu wird der Merkmalsraum in eine Menge von Punkten unterteilt, die dem Schnarchen zugeordnet werden und in den Rest, der als Nicht-Schnarchen klassifiziert wird. Um diese Klassifikation vornehmen zu können, werden diese Algorithmen zuerst anhand bekannter Schnarchgeräusche trainiert, das heißt es werden manuell Audio-Segmente mit Schnarchgeräuschen ausgewählt, die Merkmalsvektoren mᵢ davon berechnet und dann wird der Klassifikations-Algorithmus damit trainiert. Implementierungen von allen verwendeten statistischen Lernmethoden sind frei verfügbar und bereits implementiert.

Mithilfe der oben erwähnten Klassifikationsverfahren ist es möglich, festzustellen, welche Audio-Segmente S_{Ai} Schnarchgeräusche enthalten. In einem durchschnittlichen Schnarchsignal sind jedoch zwischen den lauten Phasen oft auch ruhige Zeitspannen, da oft nur beim Einatmen geschnarcht wird, wie dies in **Fig. 9** gezeigt ist. Um die gesamte Schnarchphase und auch Ruhesegmente zwischen den lauten Episoden korrekt einem Schnarchen zuordnen zu können, werden an der i-ten Stelle mehrere Segmente vorher und nachher betrachtet. Sind in dieser Menge mehr Segmente mit Schnarchgeräuschen als ein gewisser Schwellwert, so wird zum Zeitpunkt T_{audio,i} Schnarchen detektiert.

Das Ziel, aufgenommene Geräusche zu klassifizieren, um Atemgeräusche wie Schnarchen und Atmen von anderen eventuell auftretenden Hintergrundgeräuschen unterscheiden zu können, kann mit aus dem Stand der Technik bekannten Methoden gelöst werden. Solche Verfahren sind insbesondere aus den folgenden Veröffentlichungen bekannt:
Hisham Alshaer, Aditya Pandya, T Douglas Bradley, and Frank Rudzicz. Subject independent identification of breath sounds components using multiple classifiers. In Acoustics, Speech and Signal Processing (ICASSP), 2014 IEEE International Conference on, pages 3577-3581. IEEE, 2014.

Es werden verschiedene statistische Lernmethoden verwendet, um die aufgenommenen Geräusche in verschiedene Kategorien einteilen zu können. Im ersten Paper wird das Mikrofon in einer Maske am Gesicht des Patienten fixiert, dadurch gibt es kaum Hintergrundgeräusche, bzw. sind diese vernachlässigbar. In dieser Publikation werden die aufgenommenen Geräusche in einatmen, ausatmen, schnarchen und keuchen eingeteilt. Es wird dazu eine Vielzahl an verschiedenen Merkmalen generiert, mit denen die gewählte Lernmethode arbeitet.

M Cavusoglu, M Kamasak, O Erogul, T Ciloglu, Y Serinagaoglu, and T Akcam. An efficient method for snore/nonsnore classification of sleep sounds. Physiological measurement, 28(8):841, 2007.

Auch in dieser Veröffentlichung werden statistische Lernmethoden verwendet, um die aufgenommenen Geräusche in verschiedene Kategorien einteilen zu können. Das Mikrofon ist ca. 15cm vom Kopf der Testperson entfernt und die aufgenommenen Geräusche werden in "Schnarchen" bzw. "Nicht Schnarchen" unterteilt.

In **Fig. 9** sind Schnarchgeräusche Xₛ beim Ausatmen dargestellt, wobei zwischen diesen Schnarchgeräuschen Xₛ auch Hintergrundgeräusche X_{H} aufgenommen werden. Aufgrund der dargestellten Klassifikation kann jedem der Audio-Segmente S_{Ai} jeweils ein Klassifikationswert Kₐ zugeordnet werden, der im obersten Bereich der **Fig. 9** dargestellt ist.

Optional können je nach Qualität des Audio-Signals und Levels der Hintergrundgeräusche die Schnarchgeräusche noch genauer in Unterklassen, wie z.B. pathologisch und nicht pathologisch, eingeteilt werden. Unter sehr günstigen Bedingungen kann auch Atmen als separates Geräusch klassifiziert werden und dessen Stärke ermittelt werden.

In **Fig. 9** ist das Ergebnis einer verbesserten Ausführungsform der Erfindung näher dargestellt, bei dem sowohl die Ergebnisse der Auswertung der Höhenprofile als auch die Ergebnisse der akustischen Schnarchanalyse in die Bewertung der Atmungsaktivität eingehen. Typischerweise sind die Segmente Sᵢ und die Audio-Segmente S_{Ai} miteinander nicht zeitlich korreliert.

Aus diesem Grund werden zeitgleich aufgenommene Audio-Segmente S_{Ai} und Segmente Sᵢ einander zunächst aufgrund ihres jeweiligen Aufnahmezeitpunkts zugeordnet. Für jedes Segment Sᵢ stehen typischerweise mehrere Audio-Segmente S_{Ai} zur Verfügung, die während der Zeitspanne des Segments aufgenommen wurden.

Im vorliegenden Ausführungsbeispiel der Erfindung wird nach Segmenten Sᵢ mit geringer Atemtiefe T gesucht. Den in **Fig. 9** dargestellten Daten kann entnommen werden, dass im Segment S₃ eine Atemtiefe T vorliegt, die wesentlich geringer ist als in den beiden vorangehenden Segmenten S₁, S₂. Anschließend wird in allen diesem Segment S₃ zugeordneten Audio-Segmenten S_{Ai} nach fehlenden Atemgeräuschen gesucht. Hier kann festgestellt werden, dass sich in den Audio-Segmenten S_{Ai}, die gleichzeitig mit dem Segment S₃ aufgenommen wurden, keine Schnarch- oder Atemgeräusche sondern lediglich Hintergrundgeräusche X_{H} vorhanden sind. Da also einerseits eine geringe Atemtiefe erkannt wurde und andererseits auch keine Schnarch- oder Atemgeräusche festgestellt werden konnten, ist davon auszugehen, dass die betreffende Person nicht atmet.

Aus den Daten, die aus den Audio-Signalen und Höhenprofilen extrahiert wurden, können nun zu einem beliebigen Zeitpunkt Aussagen über die Atmung getroffen werden. Dazu wird wie folgt vorgegangen: Es werden zwei Indizes I und J gesucht, sodass die jeweiligen Beginnzeitpunkte T_{audio,I} der Audio-Segmente S_{Ai} und die Beginnzeitpunkte T_{3D,J} der Segmente S jeweils am nächsten beim Zeitpunkt t sind unter allen möglichen Zeitpunkten, die für die Audiosegmentierung und für die Segmentierung der Höhenprofile gewählt wurden. Die Möglichkeiten, die sich für diesen Zeitpunkt ergeben, sind in der folgenden Tabelle zusammengefasst:

| | Schnarchen bei T_{audio,I} | Kein Schnarchen bei T_{audio,I} |
|---|---|---|
| große Atemtiefe bei T_{3D,J} | Atmung | Atmung |
| keine oder geringe Atemtiefe bei T_{3D,J} | Atmung | Atemstillstand |

Im Folgenden wird eine weitere Ausführungsform der Erfindung dargestellt, bei der eine verbesserte Verknüpfung der ermittelten Atemtiefe T und der akustischen Klassifikation der Atemgeräusche und damit eine verbesserte Detektion von Atemaussetzern ermöglicht. Durch Interpolation der ermittelten Atemtiefen über die Zeit kann ein Atemtiefesignal T(t) ermittelt werden.

Diese Klassifikation beruht auf der Atemtiefefunktion T(t), die den zeitlichen Verlauf der Atemtiefe bezeichnet, sowie das Klassifikationssignal A(t), das zu jedem Zeitpunkt angibt ob und gegebenenfalls welche Atemgeräusche vorhanden waren.
Im vorliegenden Ausführungsbeispiel wird das Klassifikationssignal A(t) modifiziert und als vektorwertige Funktion festgelegt, die jedem Zeitpunkt einen Vektor zuordnet, dessen Komponenten jeweils die Intensität des jeweils erkannten Signals angeben. Konkret könnte das Klassifikationssignal A(t) Komponenten aufweisen, von denen eine die Stärke Stärke des Schnarchgeräuschs oder eines Schnarchgeräuschs von einem bestimmten Typ repräsentieren, während eine andere die Stärke der zum jeweiligen Zeitpunkt vorhandenen Hintergrundgeräusche repräsentiert.

Es besteht dann die Möglichkeit, mehrere Audio-Segmente zu einem weiteren Audio-Segment mit einer Länge von beispielsweise 5 bis 10 Sekunden zusammenzufassen und mithilfe einer Mittelwertbildung die jeweiligen Signalstärken in diesem weiteren Audio-Segment zu ermitteln. Durch diesen Schritt ist es möglich, längere Perioden, in denen eine Person beim Einatmen schnarcht, jedoch leise ausatmet, insgesamt als Schnarchen zu klassifizieren. Durch diese Mittelung wird ein gemitteltes Klassifikationssignal B(t) erstellt. Besonders vorteilhaft ist es, wenn das gemitteltes Klassifikationssignal B(t) und das Atemtiefesignal A(t) auf zeitlich miteinander übereinstimmenden Segmenten bzw. weiteren Audio-Segmenten festgelegt werden.

In einem nachfolgenden Schritt wird sowohl im Atemtiefesignal T(t) als auch im weiteren Klassifikationssignal B(t) nach Zeitpunkten gesucht, zu denen in den beiden Signalen T(t), B(t) starke Änderungen auftreten. Hierbei handelt es sich beispielsweise um Zeitpunkte, zu denen Atemgeräusche verschwinden bzw. wieder einsetzen oder Atembewegungen verschwinden oder wieder einsetzen.

Die so erkannten Zeitpunkte werden als umso relevanter eingestuft, je stärker die Änderung des Signals T(t), B(t) zum jeweiligen Zeitpunkt ist. Es wird ein diesbezüglicher Relevanzwert REL erstellt, das die Relevanz der einzelnen Zeitpunkte für den Beginn oder das Ende einer Phase mit geringer Atmung angibt.

Es werden jene Punkte als Startpunkte R_{S} oder Endpunkte R_{E} eines Atemstillstandes auswählt, für welche der Betrag des Relevanzmaßes REL über dem Betrag eines vorgegebenen Schwellwerts liegt. Ein Startzeitpunkt R_{S} kann angenommen werden, wenn der Relevanzwert REL negativ ist, ein Endzeitpunkt R_{E} kann angenommen werden, wenn der Relevanzwert REL positiv ist. Der Zeitraum AP zwischen dem Startzeitpunkt R_{S} und dem Endzeitpunkt R_{E} wird als Zeitraum geringer oder fehlender Atemtätigkeit angesehen.

Der Schwellenwert kann insbesondere schleifend bzw. gleitend gebildet werden, indem über einen Zeitbereich, insbesondere vor und/oder nach dem Vergleichszeitpunkt mit dem Referenzmaß ein Mittelwert des Referenzmaßes gebildet wird und der Schwellenwert im Bereich zwischen 120% und 200% dieses Mittelwerts festgelegt wird.

Insgesamt ist es im Rahmen der Erfindung möglich, den gesamten Schlaf einer Person über mehrere Stunden aufzuzeichnen und die Höhenprofile, gegebenenfalls auch die Audio-Signale, in einzelne Segmente, und gegebenenfalls auch Audio-Segmente, zu unterteilen. Selbstverständlich ist es auch möglich, nur einzelne besonders relevante Schlafphasen der Person zu betrachten und nur einige der aufgenommenen Höhenprofile bzw. Audio-Abtastwerte einzelnen Segmente und gegebenenfalls Audio-Segmenten zuzuordnen.

Weiters ist es möglich, die Anzahl und Dauer der ermittelten Phasen von geringer Atemtiefe oder fehlender Atemtätigkeit über den gesamten Schlaf aufzuzeichnen und statistischen Analysen zu unterziehen.

Im Folgenden werden weitere bevorzugte Ausführungsvarianten der Erfindung näher dargestellt. Grundsätzlich werden bei diesen Ausführungsvarianten zunächst Zeitbereiche ermittelt, die in für die Atmung relevanten Bereichen frei von Bewegungen sind, die nicht auf die Atmung zurückführbar sind und von anderen Bewegungen der Person während des Schlafs herrühren. Anschließend werden Bereiche des Höhenprofils identifiziert, in denen die Atmung der Person am besten zu ermitteln ist. Diese Bereiche werden anschließend verwendet, um ein Atemtiefesignal zu erstellen.

Während des Schlafs kommt es gelegentlich zu Oberkörperbewegungen der schlafenden Person wie beispielsweise Rollbewegungen. Solche Bewegungen können einfach identifiziert werden, da diese Bewegungen wesentlich stärker ausgeprägt sind als Atembewegungen, die nur eine geringe Bewegungsamplitude aufweisen und darüber hinaus weitaus regelmäßiger sind. Um zu vermeiden, dass derartige Bewegungen mit größerer Amplitude in den ermittelten Höhenprofilen zu sprunghaften Änderungen der ermittelten Signale kommen, werden Zeitbereiche zwischen den einzelnen Bewegungen identifiziert; Atemtiefemessungen werden für diese Zeitbereiche separat ermittelt. Jedem auf diese Weise gewonnenen Zeitbereich wird jeweils ein separater Atemtiefeverlauf zugeordnet. Dabei kann auf das vorstehend genannte erfindungsgemäße Vorgehen zurückgegriffen werden.

Die ermittelten Zeitbereiche, die frei von größeren Bewegungen der Person sind, werden in einzelne Segmente unterteilt, die jeweils eine Anzahl von zu aufeinander folgenden Aufnahmezeitpunkten t1, ..., tp, insbesondere innerhalb eines Zeitbereichs von 3 bis 20 Sekunden, aufgenommenen Höhenprofilen H aufweisen.

Die Auswahl des Betrachtungsbereichs 22 kann vorteilhaft vorgenommen werden, indem zunächst für sämtliche Punkte des Höhenprofils H, insbesondere für alle Einträge der Matrixdatenstruktur oder der Punktwolke, separat Zeitsignale für die Segmente des Zeitbereichs erstellt werden, das den zeitlichen Verlauf des Höhenprofils H im jeweiligen Punkt innerhalb des jeweiligen Segments angeben.

Aus jedem dieser Zeitsignale wird jeweils ein die Atmung bzw. Stärke der Atmung charakterisierender Wert abgeleitet und dem jeweiligen Punkt des Höhenprofils zugewiesen. Die Ableitung des Atemtiefewerts kann auf unterschiedliche Weise erfolgen. Neben der Verwendung der Varianz können auch spezifische Anteile des ermittelten Signalspektrums vorteilhaft zur Ermittlung der Atemtiefe herangezogen werden.

Hierfür wird jeweils für jeden Eintrag des Höhenprofils separat die Signalenergie innerhalb zweier vorgegebener Frequenzbereiche ermittelt. Der erste Frequenzbereich, mit dem sich das Vorhandensein und die Stärke der Atmung gut abschätzen lässt und der für die Stärke der Atmung charakteristisch ist, liegt etwa zwischen 10/min und 40/min. Allgemein kann die untere Schranke dieses ersten Frequenzbereichs vorteilhaft zwischen 5/min und 15/min liegen, die obere Schranke des ersten Frequenzbereichs liegt vorteilhaft zwischen 25/min und 60/min.

Diese angegebenen Grenzen sind jedoch nur exemplarisch und hängen sehr stark vom Alter des jeweiligen Patienten ab. Säuglinge atmen beispielsweise drei mal so schnell wie erwachsene Personen. Grundsätzlich ergeben sich diese Grenzen bzw. Schwellenwerte aus dem untersten angenommenen Wert für die Atemfrequenz, der bei erwachsenen Personen bei ca 10/min liegt. Die obere Grenze kann auf das dreifache bis vierfache des unteren Grenzwerts, dh auf 30/min bis 40/min festgelegt werden, insbesondere, um auch Oberschwingungen zu berücksichtigen der Atembewegungen zu berücksichtigen.

Ebenso wird ein Signalrauschen ermittelt, das durch Signalanteile innerhalb eines zweiten Frequenzbereichs feststellbar ist, der insbesondere zwischen 360/min und 900/min liegt. Allgemein kann die untere Schranke dieses zweiten Frequenzbereichs vorteilhaft zwischen 180/min und 500/min liegen, die obere Schranke des zweiten Frequenzbereichs liegt vorteilhaft zwischen 600/min und 1200/min. Auch die Festlegung dieses Frequenzbereichs ist stark abhängig von dem untersuchten Patienten. Grundsätzlich wird ein Frequenzband herangezogen, das oberhalb des ersten Frequenzbereichs liegt und auf das die Atmung des Patienten keinen Einfluss hat.

Anschließend wird von der Verarbeitungseinheit 50 der Quotient aus der Signalenergie im ersten Frequenzbereich und der Energie des Messrauschens im zweiten Frequenzbereich ermittelt, der als Atemstärkewert zur Charakterisierung des Vorhandenseins von Atmung herangezogen wird.

Anschließend wird für jedes der Segmente jeweils ein Bereich oder mehrere Bereiche 22a, 22b mit jeweils zusammenhängenden Punkten des Höhenprofils H, deren jeweiliger Atemtiefewert oberhalb eines unteren Schwellenwerts oder innerhalb eines vorgegebenen Intervalls liegen, als Betrachtungsbereich 22 ausgewählt. Bevorzugt kann dies erfolgen, indem zusammenhängende Bereiche 22a, 22b von Punkten als Betrachtungsbereich ausgewählt werden, deren Größe eine vorgegebene Anzahl von Punkten übersteigt.

Ein Bereich kann dann als zusammenhängend angesehen werden, wenn jeder Punkt des Bereichs ausgehend von einem anderen Punkt innerhalb dieses Bereichs über jeweils benachbarte Pixel erreichbar ist. Punkte des Höhenprofils H können bevorzugt dann als benachbart angesehen werden,
- wenn sie durch benachbarte Einträge der Matrix-Datenstruktur festgelegt sind oder
- wenn sie oder ihre Projektion auf eine horizontale Ebene im Raum einen einen Schwellenwert unterschreitenden Abstand voneinander aufweisen.

Die Festlegung des Zusammenhangs kann auf unterschiedliche Weise erfolgen, insbesondere ist es möglich, zur Festlegung der Nachbarschaft innerhalb des Höhenprofils diejenigen Punkte als Nachbarn festzulegen, die in höchstens einem Index maximal den Wert 1 voneinander abweichen. Dies wird auch als Vierer-Nachbarschaft bezeichnet.

Die Festlegung des jeweiligen Schwellenwerts kann dynamisch erfolgen, was eine Anpassung an unterschiedliche Schlafhaltungen ermöglich. Der Schwellenwert wird angepasst, wenn Pixelanzahl des größten zusammenhängenden Bereichs nicht innerhalb eines vorgegebenen Schwellenwertbereichs liegt. Ist die Pixelanzahl des größten Bereichs größer als der Maximalwert des Schwellenwertbereichs, wird der Schwellenwert verdoppelt, ist die Pixelanzahl kleiner als der Minimalwert des Schwellenwertbereichs, wird der Schwellenwert halbiert.

Für jedes zeitliche Segment wird jeweils der Bereich 22 mit der größten Pixelanzahl ausgewählt. Alternativ kann auch eine Anzahl N der größten Bereiche 22 ausgewählt werden. Für jedes zeitliche Segment wird jeweils der Schwerpunkt des Bereichs 22 abgespeichert. Innerhalb des Zeitbereichs wird anschließend über alle verfügbaren x-Koordinaten von Schwerpunkten jeweils der Median mx gebildet. Ebenso wird innerhalb des Zeitbereichs über alle verfügbaren y-Koordinaten von Schwerpunkten jeweils der Median my gebildet. Anschließend wird unter allen für den Zeitbereich ermittelten Bereichen 22 derjenige Bereich ermittelt, dessen Schwerpunkt den geringsten Abstand von demjenigen Punkt aufweist, dessen y-Koordinate dem Median my der y-Koordinaten entspricht und dessen x-Koordinate dem Median mx der x-Koordinaten entspricht.

Dieser Bereich wird anschließend zur Ermittlung des Atemtiefesignals für den gesamten Zeitbereich herangezogen. Das Atemtiefesignal wird ermittelt, indem für jeden Zeitpunkt jeweils separat der Mittelwert oder die Summe der Werte des Höhenprofils H innerhalb dieses Bereichs ermittelt wird. Das so ermittelte Atemtiefesignal kann allenfalls noch einer Tiefpassfilterung unterzogen werden, wobei eine Filterfrequenz zwischen 25/min und 60/min verwendet wird. Auch bei der Festlegung der Filter- oder Grenzfrequenz des Tiefpassfilters sind die genauen Werte stark vom Alter des Patienten abhängig. Neben einer Tiefpassfilterung können auch noch de-trending bzw. Hochpassfilterung eingesetzt werden.

## Patentansprüche

1. Verfahren zur Bestimmung des zeitlichen Verlaufs der Atemtiefe (T) einer, insbesondere schlafenden, Person, wobei mit einer auf die Person (1) gerichteten Detektoreinheit (20) laufend zu aufeinander folgenden Aufnahmezeitpunkten (t₁, ..., tₚ) jeweils ein Höhenprofil (H) der Person (1) erstellt wird,
a) wobei im Höhenprofil (H) eine Anzahl von zumindest zwei Punkten im Raum festgelegt ist, die auf der Oberfläche der Person (1) oder auf der Oberfläche eines auf oder neben der Person (1) befindlichen Gegenstands liegen,
b) wobei für jeden der Aufnahmezeitpunkte (t₁, ..., tₚ) das jeweilige Höhenprofil (H) in einer Datenstruktur abgespeichert und zur Verfügung gehalten wird,
c) wobei eine Anzahl von zu aufeinander folgenden Aufnahmezeitpunkten (t₁, ..., tₚ), insbesondere innerhalb eines Zeitbereichs von 3 bis 20 Sekunden, aufgenommenen Höhenprofilen (H) zu einem Segment (S₁, ..., S_{q}) zusammengefasst wird,
d) wobei ein Bereich, der den Bauch- oder Brustbereich der Person in Abhängigkeit vom jeweiligen Referenzpunkt oder Referenzbereich (21) angibt als Betrachtungsbereich (22) ausgewählt wird,
e) wobei für jedes Höhenprofil (H) innerhalb des Segments (S₁, ..., S_{q}) jeweils gesondert der Mittelwert der Abstände der innerhalb des Betrachtungsbereichs (22) befindlichen Punkte des Höhenprofils (H) zu einem Referenzpunkt oder Referenzgegenstand ermittelt wird, und für das Segment (S₁, ..., S_{q}) ein Signal (sᵢ) ermittelt wird, dem zum jeweiligen Aufnahmezeitpunkt (t₁, ..., tₚ) des Höhenprofils (H) der für dieses Höhenprofil (H) ermittelte Mittelwert zugeordnet wird, und
f) wobei basierend auf dem ermittelten Signal (sᵢ), insbesondere basierend auf dessen Signalamplitude, ein oder mehrere den zeitlichen Verlauf der Atemtiefe (T) charakterisierende Werte ermittelt wird oder werden **dadurch gekennzeichnet, dass**
g) gleichzeitig parallel zur Aufnahme des Abstands der Person von der Detektoreinheit (20) der von der Person abgegebene Schall ermittelt und in Form eines Audio-Signals (sₐ) mit einer Anzahl von Audio-Abtastwerten zur Verfügung gehalten wird,
h) das Audio-Signal (sₐ) in eine Anzahl von Audio-Segmente (S_{A1}, ..., S_{Ar}), insbesondere mit einer Länge von 100 ms bis 1 Sekunde, unterteilt wird und für jedes der Audio-Segmente (S_{A1}, ..., Sₐᵣ) untersucht wird, ob in diesem menschliche Atemgeräusche oder andere Geräusche enthalten sind, und für jedes Audio-Segment (S_{A1}, ..., S_{Ar}) jeweils ein Klassifikationsergebnis zur Verfügung gehalten wird,
i) zeitgleich aufgenommene Audio-Segmente (S_{A1}, ..., Sₐᵣ) und Segmente (S₁, ..., S_{q}) einander zugeordnet werden, und
j) nach Audio-Segmenten (S_{A1}, ..., Sₐᵣ) oder Segmenten (S₁, ..., S_{q}) mit geringer Atemtiefe oder mit fehlenden Atemgeräuschen gesucht wird und die einem solchen Audio-Segment (S_{A1}, ..., Sₐᵣ) oder Segment (S₁, ..., S_{q}) zugeordneten Segmente (S₁, ..., S_{q}) oder Audio-Segmente (S_{A1}, ..., Sₐᵣ) ebenfalls auf das Vorliegen geringer Atemtiefe oder fehlender Atemgeräuschen untersucht werden, und
k) für den Fall, dass in einander zugeordneten Segmenten (S₁, ..., S_{q}) und Audio-Segmenten (S₁, ..., S_{q}) jeweils geringe Atemtiefe und fehlender Atemgeräusche detektiert wurden, das Fehlen der Atmung der Person festgestellt wird.

2. Verfahren zur Bestimmung des zeitlichen Verlaufs der Atemtiefe (T) einer, insbesondere schlafenden, Person, wobei mit einer auf die Person (1) gerichteten Detektoreinheit (20) laufend zu aufeinander folgenden Aufnahmezeitpunkten (t₁, ..., tₚ) jeweils ein Höhenprofil (H) der Person (1) erstellt wird,
a) wobei das Höhenprofil (H) eine Anzahl von zumindest zwei Abstandswerten (d₁, ..., dₙ) zur Festlegung jeweils eines Punktes im Raum aufweist, wobei die einzelnen Abstandswerte jeweils den Abstand des Schnittpunktes eines relativ zur Detektoreinheit vorab festgelegten, insbesondere von der Detektoreinheit ausgehenden, Strahls mit der Oberfläche der Person (1) oder der Oberfläche eines auf der oder neben der Person (1) befindlichen Gegenstands von einem Referenzpunkt oder einer Referenzebene (21) angeben
b) wobei für jeden der Aufnahmezeitpunkte (t₁, ..., tₚ) jeweils eine Datenstruktur erstellt wird, die das jeweilige Höhenprofil (H) enthält, wobei sämtliche so erstellte Datenstrukturen jeweils dieselbe Größe aufweisen und jeweils mit Speicherpositionen für die einzelnen Abstandswerte des Höhenprofils (H) aufweisen,
c) wobei eine Anzahl von zu aufeinander folgenden Aufnahmezeitpunkten (t₁, ..., tₚ), insbesondere innerhalb eines Zeitbereichs von 3 bis 20 Sekunden, aufgenommenen Höhenprofilen (H) zu einem Segment (S₁, ..., S_{q}) zusammengefasst wird,
d) wobei eine Anzahl von Speicherpositionen der Datenstruktur, in denen Abstandswerte (d₁, ..., dₙ) abgespeichert sind, die den Abstand des Bauch- oder Brustbereichs der Person in Abhängigkeit vom jeweiligen Referenzpunkt oder Referenzbereich (21) angeben, als Betrachtungsbereich (22) ausgewählt wird,
e) wobei für jedes Höhenprofil (H) innerhalb eines Segments (S₁, ..., S_{q}) jeweils gesondert der Mittelwert der innerhalb des Betrachtungsbereichs (22) befindlichen Abstandswerte (d₁, ..., dₙ) ermittelt wird, und für das Segment (S₁, ..., S_{q}) ein Signal (sᵢ) ermittelt wird, dem zum jeweiligen Aufnahmezeitpunkt (t₁, ..., tₚ) des Höhenprofils (H) der für dieses Höhenprofil (H) ermittelte Mittelwert zugeordnet wird, und
f) wobei basierend auf dem ermittelten Signal (sᵢ), insbesondere basierend auf dessen Signalamplitude, ein oder mehrere den zeitlichen Verlauf der Atemtiefe (T) charakterisierende Werte ermittelt wird oder werden **dadurch gekennzeichnet, dass**
g) gleichzeitig parallel zur Aufnahme des Abstands der Person von der Detektoreinheit (20) der von der Person abgegebene Schall ermittelt und in Form eines Audio-Signals (sₐ) mit einer Anzahl von Audio-Abtastwerten zur Verfügung gehalten wird,
h) das Audio-Signal (sₐ) in eine Anzahl von Audio-Segmente (S_{A1}, ..., S_{Ar}), insbesondere mit einer Länge von 100 ms bis 1 Sekunde, unterteilt wird und für jedes der Audio-Segmente (S_{A1}, ..., Sₐᵣ) untersucht wird, ob in diesem menschliche Atemgeräusche oder andere Geräusche enthalten sind, und für jedes Audio-Segment (S_{A1}, ..., S_{Ar}) jeweils ein Klassifikationsergebnis zur Verfügung gehalten wird,
i) zeitgleich aufgenommene Audio-Segmente (S_{A1}, ..., Sₐᵣ) und Segmente (S₁, ..., S_{q}) einander zugeordnet werden, und
j) nach Audio-Segmenten (S_{A1}, ..., Sₐᵣ) oder Segmenten (S₁, ..., S_{q}) mit geringer Atemtiefe oder mit fehlenden Atemgeräuschen gesucht wird und die einem solchen Audio-Segment (S_{A1}, ..., Sₐᵣ) oder Segment (S₁, ..., S_{q}) zugeordneten Segmente (S₁, ..., S_{q}) oder Audio-Segmente (S_{A1}, ..., Sₐᵣ) ebenfalls auf das Vorliegen geringer Atemtiefe oder fehlender Atemgeräuschen untersucht werden, und
k) für den Fall, dass in einander zugeordneten Segmenten (S₁, ..., S_{q}) und Audio-Segmenten (S₁, ..., S_{q}) jeweils geringe Atemtiefe und fehlender Atemgeräusche detektiert wurden, das Fehlen der Atmung der Person festgestellt wird.

3. Verfahren nach einem der vorangehenden Ansprüche, **dadurch gekennzeichnet,**
a) **dass** für eine Anzahl von überlappenden oder nicht überlappenden Segmenten (S₁, ..., S_{q}) jeweils separat die Atemtiefe (T) ermittelt wird und/oder
b) **dass** der Betrachtungsbereich (22) für jedes Segment (S₁, ..., S_{q}) separat, insbesondere auf Grundlage des für das jeweils vorangehende Segment (S₁, ..., S_{q}) ermittelten Betrachtungsbereichs (22), ermittelt wird.

4. Verfahren nach einem der vorangehenden Ansprüche, **dadurch gekennzeichnet, dass**
- das Höhenprofil (H) durch eine zweidimensionale Matrixdatenstruktur umfassend eine Anzahl von Zeilen und Spalten charakterisiert wird,
- eine Anzahl von in Zeilen und Spalten rasterförmig angeordneten Positionen vorgegeben wird, an denen jeweils die Abstandswerte (d₁, ..., dₙ) des Höhenprofils (H) bestimmt werden,
- die Abstandswerte insbesondere festgelegt werden:
a) entlang von vorgegebenen Strahlen, die von einer Detektoreinheit ausgehen, oder
b) als die normalen Abstände von dem jeweiligen Schnittpunkt des Strahls mit der Oberfläche zur einer Referenzebene (21) mithilfe des gemessenen Abstandes und des jeweils verwendeten Messwinkels,
- wobei vorzugsweise die Messwinkel der verschiedenen Strahlen dabei derart gewählt werden, dass sich bei Auftreffen der Strahlen auf eine Ebene, die parallel zur Referenzebene (21) liegen, eine rasterförmige Anordnung ergibt,- die Matrixdatenstruktur ein Raster von derselben Größe und Struktur aufweist, und
- die Matrixdatenstruktur erstellt wird, indem die an den jeweiligen Positionen aufgenommenen Abstandswerte (d₁, ..., dₙ) an den den Positionen im Raster entsprechenden Speicherpositionen in der Matrixdatenstruktur abgespeichert und zur Verfügung gehalten werden, wobei insbesondere
a) die Matrixdatenstruktur nach ihrer Erstellung in ihren Abmessungen durch eine reduzierte Matrixdatenstruktur ersetzt wird, wobei für rechteckige, insbesondere die gesamte Matrixdatenstruktur abdeckende, und nicht überlappende Bildbereiche von jeweils gleicher größe in der Matrixdatenstruktur jeweils ein mittlerer Abstandswert (d') ermittelt wird und dieser mittlere Abstandswert (d') dem bezüglich seiner Lage entsprechenden Bildpunkt der reduzierten Matrixdatenstruktur zugeordnet wird, oder
b) die Matrixdatenstruktur nach ihrer Erstellung in ihren Abmessungen durch eine reduzierte Matrixdatenstruktur erstellt wird, deren räumliche Auflösung verringert wird, indem mehrere Einträge der Matrixdatenstruktur zu einem Eintrag der reduzierten Datenmatrixstruktur zusammengefasst werden,
wobei insbesondere nur einzelne der Abstandsmesswerte (d) für die Bildung der reduzierten Matrixdatenstruktur herangezogen werden und die übrigen Abstandsmesswerte verworfen werden,
wobei vorzugsweise die Parameter (a, b) als ganzzahlige Werte bestimmt werden und die reduzierte Matrixdatenstruktur gemäß Hᵣ(x,y,t)=H(ax,by,t) bestimmt wird, oder
c) die Matrixdatenstruktur nach ihrer Erstellung in ihren Abmessungen durch eine reduzierte Matrixdatenstruktur ersetzt wird, wobei für Bereiche der Matrixdatenstruktur jeweils ein, insbesondere mittlerer, Abstandswert (d') ermittelt wird und dieser Abstandswert (d') dem bezüglich seiner Lage entsprechenden Bildpunkte der reduzierten Datenmatrixstruktur zugeordnet wird.

5. Verfahren nach einem der vorangehenden Ansprüche, **dadurch gekennzeichnet, dass** der Betrachtungsbereich (22) gesetzt wird, indem
a) für sämtliche Punkte des Höhenprofils (H), insbesondere für alle Einträge der Matrixdatenstruktur oder der Punktwolke, separat ein Zeitsignal für das Zeitintervall erstellt wird, das den zeitlichen Verlauf des Höhenprofils (H) im jeweiligen Punkt angibt, und aus diesem Zeitsignal jeweils ein die Atemtiefe charakterisierender Atemstärkewert abgeleitet wird dem jeweiligen Punkt des Höhenprofils zugewiesen wird,
b) ein Bereich oder mehrere Bereiche (22a, 22b) mit jeweils zusammenhängenden Punkten des Höhenprofils (H), deren jeweiliger Atemstärkewert oberhalb eines unteren Schwellenwerts oder innerhalb eines vorgegebenen Intervalls liegen, als Betrachtungsbereich (22) ausgewählt wird oder werden, insbesondere
- indem zusammenhängende Bereiche (22a, 22b) von Punkten als Betrachtungsbereich ausgewählt werden, deren Größe eine vorgegebene Anzahl von Punkten übersteigt
- wobei insbesondere ein Bereich dann als zusammenhängend angesehen wird, wenn jeder Punkt des Bereichs ausgehend von einem anderen Punkt innerhalb dieses Bereichs über jeweils benachbarte Pixel erreichbar ist,
- wobei vorzugsweise Punkte des Höhenprofils (H) dann als benachbart angesehen werden,
- wenn sie durch benachbarte Einträge der Matrix-Datenstruktur festgelegt sind oder
- wenn sie oder ihre Projektion auf eine horizontale Ebene im Raum einen einen Schwellenwert unterschreitenden Abstand voneinander aufweisen,
und indem zur Erstellung des Atemstärkewerts ermittelt wird, wie hoch die Signalenergie in einem ersten Frequenzbereich, insbesondere zwischen 10/min und 40/min, ist, und ermittelt wird, wie hoch die Signalenergie in einem zweiten Frequenzbereich, insbesondere zwischen 360/min und 900/min, ist, und ein Quotient aus der Signalenergie im ersten Frequenzbereich und der Signalenergie im zweiten Frequenzbereich ermittelt wird, der als Atemstärkewert herangezogen wird.

6. Verfahren nach einem der vorangehenden Ansprüche, **dadurch gekennzeichnet, dass**
a) ein Klassifikationssignal (A(t)) erstellt wird, das für jedes Audio-Segment (S_{Ai}) jeweils zumindest ein Klassifikationsergebnis enthält, das die Art und Stärke des jeweiligen Geräuschs im Zeitbereich des jeweiligen Audio-Segment (S_{Ai}) angibt,
b) dass jeweils eine Anzahl von aufeinander folgenden Audio-Segmente (S_{Ai}) zu weiteren Audio-Segmenten (S_{Ai}), insbesondere mit einer Dauer von 5 bis 10 Sekunden zusammengefasst werden, die vorzugsweise dieselben Zeitbereiche umfassen wie die Segmente (S₁, ..., S_{q}),
c) über die innerhalb eines weiteren Audio-Segments (S_{Ai}) enthaltenen Klassifikationsergebnisse eine Mittelung vorgenommen wird und dem weiteren Audio-Segment (S_{Ai}) der jeweilige Mittelwert zugewiesen wird,
d) ein weiteres Klassifikationssignal (B(t)) durch Interpolation der Mittelwerte der weiteren Audio-Segmente (S_{Ai}) erstellt wird,
e) im weiteren Klassifikationssignal B(t) sowie im Atemtiefesignal T(t) nach Zeitpunkten gesucht wird, zu denen in den beiden Signalen (T(t), B(t)) starke Änderungen auftreten,
f) die so erkannten Zeitpunkte werden als umso relevanter eingestuft werden, je stärker die Änderung des jeweiligen Signals zum jeweiligen Zeitpunkt ist,
g) diesen Zeitpunkten jeweils ein diesbezügliches Relevanzwert zugeordnet wird, und
h) jene Zeitpunkte als Startpunkte (R_{S}) oder Endpunkte (R_{E}) eines Atemstillstandes detektiert werden, für welche der Betrag des Relevanzwerts (REL) über dem Betrag eines Schwellwert liegt, wobei insbesondere
- der Schwellenwert gebildet wird, indem über einen Zeitbereich, insbesondere vor und/oder nach dem Vergleichszeitpunkt mit dem Referenzmaß ein Mittelwert des Referenzmaßes gebildet wird und der Schwellenwert im Bereich zwischen 120% und 200% dieses Mittelwerts festgelegt wird.

7. Verfahren nach einem der vorangehenden Ansprüche, **dadurch gekennzeichnet,**
- **dass** ein Zeitbereich oder mehrere Zeitbereiche ermittelt werden, in denen die Änderung des Höhenprofils (H), insbesondere im Mittel, einen vorgegebenen Schwellenwert nicht übersteigt, und
- **dass** zur Bestimmung des Verlaufs der Atemtiefe nur aufeinander folgende Aufnahmezeitpunkte aus demselben Zeitbereich herangezogen werden, und
- **dass** gegebenenfalls bei Vorhandensein mehrerer Zeitbereiche die Bestimmung Verlaufs der Atemtiefe für mehrere ermittelte Zeitbereiche separat erfolgt.

8. Datenträger, auf dem ein Programm zur Durchführung eines Verfahrens nach einem der vorangehenden Ansprüche in einer Vorrichtung nach einem der Ansprüche 9-15 abgespeichert ist.

9. Vorrichtung zur Bestimmung des zeitlichen Verlaufs der Atemtiefe (T) einer, insbesondere schlafenden, Person, umfassend
- eine auf eine Schlafgelegenheit, insbesondere ein Bett (10) ausrichtbare Detektoreinheit (20), die laufend zu aufeinander folgenden Aufnahmezeitpunkten (t₁, ..., tₚ) jeweils ein Höhenprofil (H) der Person (1) erstellt, wobei im Höhenprofil (H) eine Anzahl von zumindest zwei Punkten im Raum festgelegt ist, die auf der Oberfläche der Person (1) oder auf der Oberfläche eines auf oder neben der Person (1) befindlichen Gegenstands liegen,
sowie
- eine Verarbeitungseinheit (50), die
a) für jeden der Aufnahmezeitpunkte (t₁, ..., tₚ) das jeweilige Höhenprofil (H) in einer Datenstruktur abgespeichert und zur Verfügung hält,
b) eine Anzahl von zu aufeinander folgenden Aufnahmezeitpunkten (t₁, ..., tₚ), insbesondere innerhalb eines Zeitbereichs von 3 bis 20 Sekunden, aufgenommenen Höhenprofilen (H) zu einem Segment (S₁, ..., S_{q}) zusammenfasst,
c) einen Bereich, der den Bauch- oder Brustbereich der Person in Abhängigkeit vom jeweiligen Referenzpunkt oder Referenzbereich (21) angibt als Betrachtungsbereich (22) auswählt,
d) für jedes Höhenprofil (H) innerhalb des Segments (S₁, ..., S_{q}) jeweils gesondert den Mittelwert der Abstände der innerhalb des Betrachtungsbereichs (22) befindlichen Punkte des Höhenprofils (H) zu einem Referenzpunkt oder Referenzgegenstand ermittelt, und für das Segment (S₁, ..., S_{q}) ein Signal (sᵢ) ermittelt wird, dem zum jeweiligen Aufnahmezeitpunkt (t₁, ..., tₚ) des Höhenprofils (H) der für dieses Höhenprofil (H) ermittelte Mittelwert zugeordnet ist, und
e) insbesondere anschließend basierend auf diesem Signal (sᵢ) oder dessen Signalamplitude einen die Atemtiefe (T) charakterisierender Wert ermittelt, **dadurch gekennzeichnet, dass** der Verarbeitungseinheit (50) ein Mikrofon (40) vorgeschaltet ist, das gleichzeitig parallel zur Aufnahme des Abstands der Person den von der Person (1) abgegebene Schall in Form eines Audio-Signals (sₐ) an seinem Ausgang zur Verfügung hält und dieses Audio-Signal der Verarbeitungseinheit zugeführt ist, und dass die Verarbeitungseinheit
f) das Audio-Signal (sₐ) in eine Anzahl von Audio-Segmente (S_{A1}, ..., S_{Ar}), insbesondere mit einer Länge von 100 ms bis 1 Sekunde, unterteilt und für jedes der Audio-Segmente (S_{A1}, ..., Sₐᵣ) untersucht, ob in diesem menschliche Atemgeräusche oder andere Geräusche zu hören sind, und für jedes Audio-Segment (S_{A1}, ..., S_{Ar}) jeweils ein Klassifikationsergebnis zur Verfügung hält,
g) zeitgleich aufgenommene Audio-Segmente (S_{A1}, ..., Sₐᵣ) und Segmente (S₁, ..., S_{q})einander zuordnet, und
h) nach Audio-Segmenten (S_{A1}, ..., Sₐᵣ) oder Segmenten (S₁, ..., S_{q}) mit geringer Atemtiefe oder mit fehlenden Atemgeräuschen sucht und die einem solchen Audio-Segment (S_{A1}, ..., Sₐᵣ) oder Segment (S₁, ..., S_{q}) zugeordneten Segmente (S₁, ..., S_{q}) oder Audio-Segmente (S_{A1}, ..., Sₐᵣ) ebenfalls auf das Vorliegen geringer Atemtiefe oder fehlender Atemgeräuschen untersucht, und
i) für den Fall, dass in einander zugeordneten Segmenten (S₁, ..., S_{q}) und Audio-Segmenten (S₁, ..., S_{q}) jeweils geringe Atemtiefe und fehlender Atemgeräusche detektiert wurden, das Fehlen der Atmung der Person feststellt.

10. Vorrichtung zur Bestimmung des zeitlichen Verlaufs der Atemtiefe (T) einer, insbesondere schlafenden, Person, umfassend
- eine auf eine Schlafgelegenheit, insbesondere ein Bett (10), ausrichtbare Detektoreinheit (20), die laufend zu aufeinander folgenden Aufnahmezeitpunkten (t₁, ..., tₚ) jeweils ein Höhenprofil (H) der Person (1) erstellt, wobei das Höhenprofil (H) eine Anzahl von zumindest zwei Abstandswerten (d₁, ..., dₙ) zur Festlegung jeweils eines Punktes im Raum aufweist, wobei die einzelnen Abstandswerte jeweils den Abstand des Schnittpunktes eines relativ zur Detektoreinheit vorab festgelegten, insbesondere von der Detektoreinheit ausgehenden, Strahls mit der Oberfläche der Person (1) oder der Oberfläche eines auf der oder neben der Person (1) befindlichen Gegenstands von einem Referenzpunkt oder einer Referenzebene (21) angeben
sowie
- eine Verarbeitungseinheit (50), die
a) für jeden der Aufnahmezeitpunkte (t₁, ..., tₚ) jeweils eine Datenstruktur erstellt, die das jeweilige Höhenprofil (H) enthält, wobei sämtliche so erstellte Datenstrukturen jeweils dieselbe Größe aufweisen und jeweils mit Speicherpositionen für die einzelnen Abstandswerte des Höhenprofils (H) aufweisen,
b) eine Anzahl von zu aufeinander folgenden Aufnahmezeitpunkten (t₁, ..., tₚ), insbesondere innerhalb eines Zeitbereichs von 3 bis 20 Sekunden, aufgenommenen Höhenprofilen (H) zu einem Segment (S₁, ..., S_{q}) zusammenfasst,
c) eine Anzahl von Speicherpositionen der Datenstruktur, in denen Abstandswerte (d₁, ..., dₙ) abgespeichert sind, die den Abstand des Bauch- oder Brustbereichs der Person in Abhängigkeit vom jeweiligen Referenzpunkt oder Referenzbereich (21) angeben, als Betrachtungsbereich (22) auswählt,
d) für jedes Höhenprofil (H) innerhalb eines Segments (S₁, ..., S_{q}) jeweils gesondert den Mittelwert der innerhalb des Betrachtungsbereichs (22) befindlichen Abstandswerte (d₁, ..., dₙ) ermittelt, und für das Segment (S₁, ..., S_{q}) ein Signal (sᵢ) ermittelt, dem zum jeweiligen Aufnahmezeitpunkt (t₁, ..., tₚ) des Höhenprofils (H) der für dieses Höhenprofil (H) ermittelte Mittelwert zugeordnet ist,
e) insbesondere anschließend basierend auf diesem Signal (sᵢ) oder dessen Signalamplitude einen die Atemtiefe (T) charakterisierender Wert ermittelt, **dadurch gekennzeichnet, dass** der Verarbeitungseinheit (50) ein Mikrofon (40) vorgeschaltet ist, das gleichzeitig parallel zur Aufnahme des Abstands der Person den von der Person (1) abgegebene Schall in Form eines Audio-Signals (sₐ) an seinem Ausgang zur Verfügung hält und dieses Audio-Signal der Verarbeitungseinheit zugeführt ist, und dass die Verarbeitungseinheit
f) das Audio-Signal (sₐ) in eine Anzahl von Audio-Segmente (S_{A1}, ..., S_{Ar}), insbesondere mit einer Länge von 100 ms bis 1 Sekunde, unterteilt und für jedes der Audio-Segmente (S_{A1}, ..., Sₐᵣ) untersucht, ob in diesem menschliche Atemgeräusche oder andere Geräusche zu hören sind, und für jedes Audio-Segment (S_{A1}, ..., S_{Ar}) jeweils ein Klassifikationsergebnis zur Verfügung hält,
g) zeitgleich aufgenommene Audio-Segmente (S_{A1}, ..., Sₐᵣ) und Segmente (S₁, ..., S_{q})einander zuordnet, und
h) nach Audio-Segmenten (S_{A1}, ..., Sₐᵣ) oder Segmenten (S₁, ..., S_{q}) mit geringer Atemtiefe oder mit fehlenden Atemgeräuschen sucht und die einem solchen Audio-Segment (S_{A1}, ..., Sₐᵣ) oder Segment (S₁, ..., S_{q}) zugeordneten Segmente (S₁, ..., S_{q}) oder Audio-Segmente (S_{A1}, ..., Sₐᵣ) ebenfalls auf das Vorliegen geringer Atemtiefe oder fehlender Atemgeräuschen untersucht, und
i) für den Fall, dass in einander zugeordneten Segmenten (S₁, ..., S_{q}) und Audio-Segmenten (S₁, ..., S_{q}) jeweils geringe Atemtiefe und fehlender Atemgeräusche detektiert wurden, das Fehlen der Atmung der Person feststellt.

11. Vorrichtung nach einem der vorangegangenen Ansprüche, **dadurch gekennzeichnet, dass** die Verarbeitungseinheit (50)
a) für eine Anzahl von überlappenden oder nicht überlappenden Segmenten (S₁, ..., S_{q}) jeweils separat die Atemtiefe (T) ermittelt und/oder
b) den Betrachtungsbereich (22) für jedes Segment (S₁, ..., S_{q}) separat, insbesondere auf Grundlage des für das jeweils vorangehende Segment (S₁, ..., S_{q}) ermittelten Betrachtungsbereichs (22), ermittelt.

12. Vorrichtung nach einem der vorangegangenen Ansprüche, **dadurch gekennzeichnet, dass** die Verarbeitungseinheit (50)
- das Höhenprofil (H) durch eine zweidimensionale Matrixdatenstruktur umfassend eine Anzahl von Zeilen und Spalten charakterisiert,
- eine Anzahl von in Zeilen und Spalten rasterförmig angeordneten Positionen vorgegeben wird, an denen jeweils die Abstandswerte (d₁, ..., dₙ) des Höhenprofils (H) bestimmt,
- die Abstandswerte insbesondere festlegt
a) als Abstandswerte entlang von vorgegebenen Strahlen, die von einer Detektoreinheit ausgehen, oder
b) als die normalen Abstände von dem jeweiligen Schnittpunkt des Strahls mit der Oberfläche zur einer Referenzebene (21) mithilfe des gemessenen Abstandes und des jeweils verwendeten Messwinkels,
- wobei sie vorzugsweise die Messwinkel der verschiedenen Strahlen dabei derart wählt, dass sich bei Auftreffen der Strahlen auf eine Ebene, die parallel zur Referenzebene (21) liegen, eine rasterförmige Anordnung ergibt,- die Matrixdatenstruktur ein Raster von derselben Größe und Struktur aufweist, und
- die Matrixdatenstruktur erstellt, indem sie die an den jeweiligen Positionen aufgenommenen Abstandswerte (d₁, ..., dₙ) an den den Positionen im Raster entsprechenden Speicherpositionen in der Matrixdatenstruktur abspeichert und zur Verfügung hält und dass
a) die Verarbeitungseinheit (50) die Matrixdatenstruktur nach ihrer Erstellung in ihren Abmessungen durch eine reduzierte Matrixdatenstruktur ersetzt,
wobei sie für rechteckige, insbesondere die gesamte Matrixdatenstruktur abdeckende, und nicht überlappende Bildbereiche von jeweils gleicher Größe in der Matrixdatenstruktur jeweils einen mittleren Abstandswert (d') ermittelt und diesen mittleren Abstandswert (d') dem bezüglich seiner Lage entsprechenden Bildpunkt der reduzierten Matrixdatenstruktur zuordnet, oder
b) die Verarbeitungseinheit (50) die Matrixdatenstruktur nach ihrer Erstellung in ihren Abmessungen durch eine reduzierte Matrixdatenstruktur ersetzt und deren räumliche Auflösung verringert, indem sie mehrere Einträge der Matrixdatenstruktur zu einem Eintrag der reduzierten Datenmatrixstruktur zusammenfasst,
wobei sie insbesondere nur einzelne der Abstandsmesswerte (d) für die Bildung der reduzierten Matrixdatenstruktur heranzieht und die übrigen Abstandsmesswerte verwirft, wobei sie vorzugsweise die Parameter (a, b) als ganzzahlige Werte bestimmt und die reduzierte Matrixdatenstruktur gemäß Hᵣ(x,y,t)=H(ax,by,t) festlegt, oder
c) die Verarbeitungseinheit (50) die Matrixdatenstruktur nach ihrer Erstellung in ihren Abmessungen durch eine reduzierte Matrixdatenstruktur ersetzt, wobei sie für Bereiche der Matrixdatenstruktur jeweils einen, insbesondere mittleren, Abstandswert (d') ermittelt und diesen Abstandswert (d') dem bezüglich seiner Lage entsprechenden Bildpunkte der reduzierten Datenmatrixstruktur zuordnet.

13. Vorrichtung nach einem der Ansprüche 9 bis 12, **dadurch gekennzeichnet, dass** die Verarbeitungseinheit (50) den Betrachtungsbereich (22) festlegt, indem sie
a) für sämtliche Punkte des Höhenprofils (H), insbesondere für alle Einträge der Matrixdatenstruktur oder der Punktwolke, separat ein Zeitsignal für DAS Zeitintervall erstellt, das den zeitlichen Verlauf des Höhenprofils (H) im jeweiligen Punkt angibt, und aus diesem Zeitsignal jeweils ein die Atemtiefe charakterisierender Atemstärkewert ableitet und dem jeweiligen Punkt des Höhenprofils zuweist,
b) ein Bereich oder mehrere Bereiche (22a, 22b) mit jeweils zusammenhängenden Punkten des Höhenprofils (H), deren jeweiliger Atemstärkewert oberhalb eines unteren Schwellenwerts oder innerhalb eines vorgegebenen Intervalls liegen, als Betrachtungsbereich (22) auswählt, insbesondere
- indem sie zusammenhängende Bereiche (22a, 22b) von Punkten als Betrachtungsbereich auswählt, deren Größe eine vorgegebene Anzahl von Punkten übersteigt,
- wobei insbesondere ein Bereich dann als zusammenhängend gilt, wenn jeder Punkt des Bereichs ausgehend von einem anderen Punkt innerhalb dieses Bereichs über jeweils benachbarte Pixel erreichbar ist,
- wobei vorzugsweise Punkte des Höhenprofils (H) dann als benachbart gelten,
- wenn sie durch benachbarte Einträge der Matrix-Datenstruktur festgelegt sind oder
- wenn sie oder ihre Projektion auf eine horizontale Ebene im Raum einen einen Schwellenwert unterschreitenden Abstand voneinander aufweisen,
und dass die Verarbeitungseinheit (50) zur Erstellung des Atemstärkewerts ermittelt,
- wie hoch die Signalenergie in einem ersten Frequenzbereich, insbesondere zwischen 10/min und 40/min, ist, und
- wie hoch die Signalenergie in einem zweiten Frequenzbereich, insbesondere zwischen 360/min und 900/min, ist, und
dass die Verarbeitungseinheit (50) den Quotienten aus der Energie im ersten Frequenzbereich und der Energie im zweiten Frequenzbereich ermittelt, und diesen als Atemstärkewert heranzieht.

14. Vorrichtung nach einem der vorangegangenen Ansprüche, **dadurch gekennzeichnet, dass** die Verarbeitungseinheit (50)
a) ein Klassifikationssignal (A(t)) erstellt, das für jedes Audio-Segment (S_{Ai}) jeweils zumindest ein Klassifikationsergebnis enthält, das die Art und Stärke des jeweiligen Geräuschs im Zeitbereich des jeweiligen Audio-Segment (S_{Ai}) angibt,
b) jeweils eine Anzahl von aufeinander folgenden Audio-Segmente (S_{Ai}) zu weiteren Audio-Segmenten (S_{Ai}), insbesondere mit einer Dauer von 5 bis 10 Sekunden zusammengefasst, die vorzugsweise dieselben Zeitbereiche umfassen wie die Segmente (S₁, ..., S_{q}),
c) über die innerhalb eines weiteren Audio-Segments (S_{Ai}) enthaltenen Klassifikationsergebnisse eine Mittelung vornimmt und dem weiteren Audio-Segment (S_{Ai}) den jeweilige Mittelwert zuweist,
d) ein weiteres Klassifikationssignal (B(t)) durch Interpolation der Mittelwerte der weiteren Audio-Segmente (S_{Ai}) erstellt,
e) im weiteren Klassifikationssignal B(t) sowie im Atemtiefesignal T(t) nach Zeitpunkten sucht, zu denen in den beiden Signalen (T(t), B(t)) starke Änderungen auftreten, und
die so erkannten Zeitpunkte als umso relevanter eingestuft, je stärker die Änderung des jeweiligen Signals zum jeweiligen Zeitpunkt ist, wobei sie diesen Zeitpunkten jeweils ein diesbezügliches Relevanzmaß zuordnet, und
f) jene Zeitpunkten als Startpunkte (R_{S}) oder Endpunkte (R_{E}) eines Atemstillstandes detektiert, für welche der Betrag des Relevanzwerts (REL) über dem Betrag eines Schwellwert liegt, wobei sie insbesondere
- der Schwellenwert bildet, indem sie über einen Zeitbereich, insbesondere vor und/oder nach dem Vergleichszeitpunkt mit dem Referenzmaß ein Mittelwert des Referenzmaßes bildet und der Schwellenwert im Bereich zwischen 120% und 200% dieses Mittelwerts festlegt.

15. Vorrichtung nach einem der vorangegangenen Ansprüche, **dadurch gekennzeichnet**, die Verarbeitungseinheit (50)
- einen Zeitbereich oder mehrere Zeitbereiche ermittelt, in denen die Änderung des Höhenprofils (H), insbesondere im Mittel, einen vorgegebenen Schwellenwert nicht übersteigt, und
- zur Bestimmung des Verlaufs der Atemtiefe nur aufeinander folgende Aufnahmezeitpunkte aus demselben Zeitbereich heranzieht, und
- gegebenenfalls bei Vorhandensein mehrerer Zeitbereiche die Bestimmung Verlaufs der Atemtiefe für mehrere ermittelte Zeitbereiche separat vornimmt.

## Claims

1. A method for determining the time curve of the depth of breath (T) of a, particularly sleeping, person, wherein by means of a detector unit (20) being directed towards the person (1) continually at successive recording times (t₁, ..., tₚ) a height profile (H) of the person (1) is respectively created,
a) wherein in the height profile (H) a number of at least two points is set in the room, which are positioned on the surface of the person (1) or on the surface of an object located on or near to the person (1),
b) wherein for each of the recording times (t₁, ..., tₚ) the respective height profile (H) is saved in a data structure and made available,
c) wherein a number of height profiles (H) recorded at successive recording times (t₁, ..., tₚ), particularly within a time range of 3 to 20 seconds, is merged into one segment (S₁, ..., S_{q}),
d) wherein an area indicating the abdominal or chest area of the person depending on the respective reference point or reference range (21), is selected as the viewing area (22),
e) wherein respectively separately for each height profile (H) within the segment (S₁, ..., S_{q}) the average is determined of the intervals of the points of the height profile (H) being located within the viewing area (22) at a reference point or reference target, and for the segment (S₁, ..., S_{q}) a signal (sᵢ) is determined, to which the average calculated for this height profile (H) is assigned to the respective recording time (t₁, ..., tₚ) of the height profile (H), and
f) wherein based on the calculated signal (sᵢ), particularly based on its signal amplitude, one or more values characterising the time curve of the depth of breath (T) is or are calculated, **characterised in that**
g) simultaneously in parallel to the recording of the distance of the person from the detector unit (20) the noise emitted by the person is calculated and made available in the form of an audio signal (sₐ) with a number of audio samples,
h) the audio signal (sₐ) is split into a number of audio segments (S_{A1}, ..., S_{Ar}), particularly with a length of 100 ms to 1 second, and for each of the audio segments (S_{A1}, ..., Sₐᵣ) it is investigated whether human breath sounds or other noises are contained therein, and for each audio segment (S_{A1}, ..., S_{Ar}) a classification result is made available respectively,
i) simultaneously recorded audio segments (S_{A1}, ..., Sₐᵣ) and segments (S₁, ..., S_{q}) are assigned to one another, and
j) audio segments (S_{A1}, ..., Sₐᵣ) or segments (S₁, ..., S_{q}) with low depth of breath or a lack of breath sounds are searched for and the segments (S₁, ..., S_{q}) or audio segments (S_{A1}, ..., Sₐᵣ) assigned to such an audio segment (S_{A1}, ..., Sₐᵣ) or segment (S₁, ..., S_{q}) are also investigated for the presence of low depth of breath or a lack of breath sounds, and
k) in the case that low depth of breath and a lack of breath sounds were respectively detected in consecutive segments (S₁, ..., S_{q}) and audio segments (S₁, ..., S_{q}), the absence of respiration of the person is detected.

2. A method for determining the time curve of the depth of breath (T) of a, particularly sleeping, person, wherein by means of a detector unit (20) being directed towards the person (1) continually at successive recording times (t₁, ..., tₚ) a height profile (H) of the person (1) is respectively created,
a) wherein in the height profile (H) has a number of at least two distance values (d₁, ..., dₙ) to respectively determine a point in the room, wherein the individual distance values respectively indicate the distance of the intersection of a beam that has been predetermined in relation to the detector unit, particularly extending from the detector unit, with the surface of the person (1) or the surface of an object located on or near to the person (1) of a reference point or a reference plane (21),
b) wherein for each of the recording times (t₁, ..., tₚ) a data structure is respectively created containing the relevant height profile (H), wherein all data structures created such have respectively the same size and also respectively have memory locations for the individual distance values of the height profile (H),
c) wherein a number of height profiles (H) recorded at successive recording times (t₁, ..., tₚ), particularly within a time range of 3 to 20 seconds, is merged into one segment (S₁, ..., S_{q}),
d) wherein a number of memory locations of the data structure, in which distance values (d₁, ..., dₙ) are saved stating the distance of the abdomen or chest area of the person depending on the respective reference point or reference range (21), is selected as the viewing area (22),
e) wherein respectively separately for each height profile (H) within a segment (S₁, ..., S_{q}) the average of the distance values (d₁, ..., dₙ) located within the viewing area (22) is determined, and for the segment (S₁, ..., S_{q}) a signal (sᵢ) is determined, to which the average calculated for this height profile (H) is assigned to the respective recording time (t₁, ..., tₚ) of the height profile (H), and
f) wherein based on the calculated signal (sᵢ), particularly based on its signal amplitude, one or more values characterising the time curve of the depth of breath (T) is or are calculated, **characterised in that**
g) simultaneously in parallel to the recording of the distance of the person from the detector unit (20) the noise emitted by the person is calculated and made available in the form of an audio signal (sₐ) with a number of audio samples,
h) the audio signal (sₐ) is split into a number of audio segments (S_{A1}, ..., S_{Ar}), particularly with a length of 100 ms to 1 second, and for each of the audio segments (S_{A1}, ..., Sₐᵣ) it is investigated whether human breath sounds or other noises are contained therein, and for each audio segment (S_{A1}, ..., S_{Ar}) a classification result is made available respectively,
i) simultaneously recorded audio segments (S_{A1}, ..., Sₐᵣ) and segments (S₁, ..., S_{q}) are assigned to one another, and
j) audio segments (S_{A1}, ..., Sₐᵣ) or segments (S₁, ..., S_{q}) with low depth of breath or a lack of breath sounds are searched for and the segments (S₁, ..., S_{q}) or audio segments (S_{A1}, ..., Sₐᵣ) assigned to such an audio segment (S_{A1}, ..., Sₐᵣ) or segment (S₁, ..., S_{q}) are also investigated for the presence of low depth of breath or a lack of breath sounds, and
k) in the case that low depth of breath and a lack of breath sounds were respectively detected in consecutive segments (S₁, ..., S_{q}) and audio segments (S₁, ..., S_{q}), the absence of respiration of the person is detected.

3. The method according to one of the preceding claims, **characterised in that**
a) for a number of overlapping or non-overlapping segments (S₁, ..., S_{q}) the depth of breath (T) is respectively calculated and/or
b) the viewing area (22) for each segment (S₁, ..., S_{q}) is calculated separately, particularly on the basis of the viewing area (22) calculated for the respectively previous segment (S₁, ..., S_{q}).

4. The method according to one of the preceding claims, **characterised in that**
- the height profile (H) is **characterised by** a two-dimensional matrix data structure comprising a number of lines and columns,
- a number of positions being arranged in a grid-like manner in lines and columns is predefined, at which the distance values (d₁, ..., dₙ) of the height profile (H) are respectively calculated,
- the distance values are particularly defined:
a) along predefined beams emitting from a detector unit, or
b) as the standard distances from the respective intersection of the beam with the surface for a reference plane (21) with the help of the measured distance and the respectively used measurement angle,
- wherein preferably the measurement angles of the various beams are selected such that a grid-like arrangement arises upon impingement of the beams on a plane being parallel to the reference plane (21),
- the matrix data structure has a grid of the same size and structure, and
- the matrix data structure is created by saving and making available the distance values (d₁, ..., dₙ) recorded in the matrix data structure at the memory locations according to the positions in the grid, wherein particularly
a) the matrix data structure is replaced after its generation in its dimensions by a reduced matrix data structure, wherein for rectangular pictures areas, particularly covering the entire matrix data structure, and non-overlapping picture areas of respectively the same size in the matrix data structure an average distance value (d') is calculated respectively and this average distance value (d') is assigned to the respective image point of the reduced matrix data structure corresponding to its location, or
b) the matrix data structure is created after its generation in its dimensions by a reduced matrix data structure, the spatial resolution of which is reduced, by summarises several entries of the matrix data structure into one entry of the reduced data matrix structure, wherein particularly only individual distance measurements (d) are uses for the formation of the reduced matrix data structure and the other distance measurements are discarded, wherein preferably the parameters (a, b) are defined as integer values and the reduced matrix data structure is defined according to Hᵣ(x,y,t)=H(ax,by,t), or
c) the matrix data structure is replaced after its generation in its dimensions by a reduced matrix data structure, wherein for areas of the matrix data structure one, particularly average, distance measurement (d') is respectively calculated and this distance value (d') is assigned to the respective image points of the reduced matrix data structure corresponding to its location.

5. The method according to one of the preceding claims, **characterised in that** the viewing area (22) is set by
a) separately creating a time signal for the time interval for all points of the height profile (H), particularly for all entries of the matrix data structure or the scatter plot, said time signal stating the time curve of the height profile (H) in the respective point, and from this time signal a expiratory force value characterising the depth of breath is respectively derived and assigned to the respective point of the height profile,
b) an area or several areas (22a, 22b) with respectively continuous points of the height profile (H), their respective expiratory force value above a lower threshold value or within a predefined interval, is or are selected as the viewing area (22), particularly
- **in that** continuous areas (22a, 22b) of points are selected as the viewing area, the size of which exceeds a predefined number of points,
- wherein particularly one area is then considered as continuous, if each point of the area is achievable by means of the respectively neighbouring pixel based on another point within this area,
- wherein preferably points of the height profile (H) are then considered as neighbouring,
- if they are determined through neighbouring entries of the matrix data structure or
- if they or their projection on a horizontal plane in the room has a distance from one another falling below a threshold value,
and **in that** to create the expiratory force value it is calculated how high the signal energy in a first frequency range, particularly between 10/min and 40/min, is and it is calculated how high the signal energy in a second frequency range, particularly between 360/min and 900/min, and a quotient from the signal energy in the first frequency range and the signal energy in the second frequency range is calculated, which is used as the expiratory force value.

6. The method according to one of the preceding claims, **characterised in that**
a) a classification signal (A(t)) is created, which contains for each audio segment (S_{Ai}) respectively at least one classification result stating the type and strength of the relevant noise in the time range of the respective audio segment (S_{Ai}),
b) that a number of consecutive audio segments (S_{Ai}) are respectively merged with further audio segments (S_{Ai}), particularly with a duration of 5 to 10 seconds, which preferably incorporate the same time ranges as the segments (S₁, ..., S_{q}),
c) using the classification results contained within a further audio segment (S_{Ai}) an averaging is carried out and the further audio segment (S_{Ai}) is assigned to the respective average value,
d) a further classification signal (B(t)) is created by means of interpolation of the average values of the further audio segments (S_{Ai}),
e) in the further classification signal B(t) and in the depth of breath signal T(t) time points are searched, at which strong changes occur in both signals (T(t), B(t)),
f) the such identified time points are classified as more relevant, the stronger the change of the respective signal at the respective time point is,
g) a relevant value relating to this is assigned respectively to these time points, and
h) those time points are detected as the starting points (R_{S}) or end points (R_{E}) of a respiratory arrest, for which the amount of the relevance value is above the amount of a threshold value, wherein particularly
- the threshold value is formed by generating an average value of the reference measure over a time range, particularly before and/or after the comparison point with the reference measure and defining the threshold value in the range between 120% and 200% of this average value.

7. The method according to one of the preceding claims, **characterised in that**
- a time range or number of time ranges are calculated, in which the change of the height profile (H), particularly on average, does not exceed a particularly threshold value, and
- **in that** to define the progress of the depth of breath only consecutively following recording times from the same time range are used, and
- **in that** where several time ranges exist the determination of the progress of the depth of breath occurs separately for a number of calculated time ranges.

8. A data carrier on which a programme to execute a method according to one of the preceding claims is saved in a device according to one of claims 9 to 15.

9. The device to define the time curve of the depth of breath (T) of a, particularly sleeping, person, comprising
- wherein a detector unit (20) being directable towards a sleeping accommodation, particularly a bed (10), which respectively creates a height profile (H) of the person (1) continually at successive recording times (t₁, ..., tₚ), wherein in the height profile (H) a number of at least two points is set in the room, which are positioned on the surface of the person (1) or on the surface of an object located on or near to the person (1),
and
- a processing unit (50), which
a) for each of the recording times (t₁, ..., tₚ) saves and keeps available in a data structure the respective height profile (H),
b) merges a number of height profiles (H) recorded at successive recording times (t₁, ..., tₚ), particularly within a time range of 3 to 20 seconds, into one segment (S₁, ..., S_{q}),
c) selects an area indicating the abdominal or chest area of the person depending on the respective reference point or reference range (21), as the viewing area (22),
d) determines a signal (sᵢ) respectively separately for each height profile (H) within the segment (S₁, ..., S_{q}) the average is determined of the intervals of the points of the height profile (H) being located within the viewing area (22) at a reference point or reference target, and for the segment (S₁, ..., S_{q}), to which the average calculated for this height profile (H) is assigned to the respective recording time (t₁, ..., tₚ) of the height profile (H), and
e) particularly then based on this signal (sᵢ) or its signal amplitude determines a value characterising the depth of breath (T), **characterised in that** a microphone (40) is upstream of the processing unit (50), said microphone keeping available simultaneously in parallel to the recording of the distance of the person the sound emitted by the person (1) in the form of an audio signal (sₐ) to its output and this audio signal is inputted to the processing unit, and **in that** the processing unit
f) splits the audio signal (sₐ) into a number of audio segments (S_{A1}, ..., S_{Ar}), particularly with a length of 100 ms to 1 second, and investigates for each of the audio segments (S_{A1}, ..., Sₐᵣ) whether human breath sounds or other noises can be heard, and provides respectively a classification result for each audio segment (S_{A1}, ..., S_{Ar}),
g) assigns to one another simultaneously recorded audio segments (S_{A1}, ..., Sₐᵣ) and segments (S₁, ..., S_{q}), and
h) searches for audio segments (S_{A1}, ..., Sₐᵣ) or segments (S₁, ..., S_{q}) with low depth of breath or a lack of breath sounds and also investigates the segments (S₁, ..., S_{q}) or audio segments (S_{A1}, ..., Sₐᵣ) assigned to such an audio segment (S_{A1}, ..., Sₐᵣ) or segment (S₁, ..., S_{q}) for the presence of low depth of breath or a lack of breath sounds, and
i) detects in the case that low depth of breath and a lack of breath sounds were respectively detected in consecutive segments (S₁, ..., S_{q}) and audio segments (S₁, ..., S_{q}), the absence of respiration of the person.

10. The device to define the time curve of the depth of breath (T) of a, particularly sleeping, person, comprising
- wherein a detector unit (20) being directable towards a sleeping accommodation, particularly a bed (10), which respectively creates a height profile (H) of the person (1) continually at successive recording times (t₁, ..., tₚ), wherein in the height profile (H) has a number of at least two distance values (d₁, ..., dₙ) to respectively determine a point in the room, wherein the individual distance values respectively indicate the distance of the intersection of a beam that has been predetermined in relation to the detector unit, particularly extending from the detector unit, with the surface of the person (1) or the surface of an object located on or near to the person (1) of a reference point or a reference plane (21),
and
- a processing unit (50), which
a) creates for each of the recording times (t₁, ..., tₚ) a data structure containing the relevant height profile (H), wherein all data structures created such have respectively the same size and also respectively have memory locations for the individual distance values of the height profile (H),
b) merges a number of height profiles (H) recorded at successive recording times (t_{1;} ..., tₚ), particularly within a time range of 3 to 20 seconds, into one segment (S₁, ..., S_{q}),
c) selects as the viewing area (22) a number of memory locations of the data structure, in which distance values (d₁, ..., dₙ) are saved stating the distance of the abdomen or chest area of the person depending on the respective reference point or reference range (21),
d) determines respectively separately for each height profile (H) within a segment (S₁, ..., S_{q}) the average of the distance values (d₁, ..., dₙ) located within the viewing area (22), and for the segment (S₁, ..., S_{q}) determines a signal (sᵢ), to which the average calculated for this height profile (H) is assigned to the respective recording time (t₁, ..., tₚ) of the height profile (H),
e) particularly then based on this signal (sᵢ) or its signal amplitude determines a value characterising the depth of breath (T), **characterised in that** a microphone (40) is upstream of the processing unit (50), said microphone keeping available simultaneously in parallel to the recording of the distance of the person the sound emitted by the person (1) in the form of an audio signal (sₐ) to its output and this audio signal is inputted to the processing unit, and **in that** the processing unit
f) splits the audio signal (sₐ) into a number of audio segments (S_{A1}, ..., S_{Ar}), particularly with a length of 100 ms to 1 second, and investigates for each of the audio segments (S_{A1}, ..., Sₐᵣ) whether human breath sounds or other noises can be heard, and provides respectively a classification result for each audio segment (S_{A1}, ..., S_{Ar}),
g) assigns to one another simultaneously recorded audio segments (S_{A1}, ..., Sₐᵣ) and segments (S₁, ..., S_{q}), and
h) searches for audio segments (S_{A1}, ..., Sₐᵣ) or segments (S₁, ..., S_{q}) with low depth of breath or a lack of breath sounds and also investigates the segments (S₁, ..., S_{q}) or audio segments (S_{A1}, ..., Sₐᵣ) assigned to such an audio segment (S_{A1}, ..., Sₐᵣ) or segment (S₁, ..., S_{q}) for the presence of low depth of breath or a lack of breath sounds, and
i) detects in the case that low depth of breath and a lack of breath sounds were respectively detected in consecutive segments (S₁, ..., S_{q}) and audio segments (S₁, ..., S_{q}), the absence of respiration of the person.

11. The device according to one of the preceding claims, **characterised in that** the processing unit (50)
a) calculates the depth of breath (T) respectively for a number of overlapping or non-overlapping segments (S₁, ..., S_{q}) and/or
b) calculates the viewing area (22) for each segment (S₁, ..., S_{q}) separately, particularly on the basis of the viewing area (22) calculated for the respectively previous segment (S₁, ..., S_{q}).

12. The device according to one of the preceding claims, **characterised in that** the processing unit (50)
- characterises the height profile (H) by a two-dimensional matrix data structure comprising a number of lines and columns,
- a number of positions being arranged in a grid-like manner in lines and columns is predefined, on which the distance values (d₁, ..., dₙ) of the height profile (H) are respectively defined,
- particularly defines the distance values
a) as distance values along predefined beams emitting from a detector unit, or
b) as the standard distances from the respective intersection of the beam with the surface for a reference plane (21) with the help of the measured distance and the respectively used measurement angle,
- wherein it preferably selects the measurement angles of the various beams such that a grid-like arrangement arises upon impingement of the beams on a plane being parallel to the reference plane (21),
- the matrix data structure has a grid of the same size and structure, and
- creates the matrix data structure by saving and making available the distance values (d₁, ..., dₙ) recorded in the matrix data structure at the memory locations according to the positions in the grid and that
a) the processing unit (50) replaces the matrix data structure after its generation in its dimensions by a reduced matrix data structure,
wherein for rectangular pictures areas, particularly covering the entire matrix data structure, and non-overlapping picture areas of respectively the same size in the matrix data structure an average distance value (d') is calculated respectively and this average distance value (d') is assigned to the respective image point of the reduced matrix data structure corresponding to its location, or
b) the processing unit (50) replaces the matrix data structure is created after its generation in its dimensions by a reduced matrix data structure and reduces its spatial resolution, by summarising several entries of the matrix data structure into one entry of the reduced data matrix structure,
wherein it particularly only uses individual distance measurements (d) for the formation of the reduced matrix data structure and discards the other distance measurements, wherein it preferably defines the parameters (a, b) as integer values and defines the reduced matrix data structure according to Hᵣ(x,y,t)=H(ax,by,t), or
c) the processing unit (50) replaces the matrix data structure after its generation in its dimensions by a reduced matrix data structure, wherein it calculates respectively for areas of the matrix data structure one, particularly average, distance measurement (d') and assigns this distance value (d') to the respective image points of the reduced matrix data structure corresponding to its location.

13. The device according to at least one of claims 9 to 12, **characterised in that** the processing unit (50) defines the viewing area (22), by
a) separately creating for all points of the height profile (H), particularly for all entries of the matrix data structure or the scatter plot, a time signal for THE time interval, which states the time curve of the height profile (H) in the respective point, and from this: time signal derives respectively a expiratory force value characterising the depth of breath and assigns to the respective point of the height profile,
b) selects an area or several areas (22a, 22b) with respectively continuous points of the height profile (H), their respective expiratory force value above a lower threshold value or within a predefined interval, as the viewing area (22), particularly
- by selecting continuous areas (22a, 22b) of points as the viewing area, the size of which exceeds a predefined number of points,
- wherein particularly one area is then considered as continuous, if each point of the area is achievable by means of the respectively neighbouring pixel based on another point within this area,
- wherein preferably points of the height profile (H) are then considered as neighbouring,
- if they are determined through neighbouring entries of the matrix data structure or
- if they or their projection on a horizontal plane in the room has a distance from one another falling below a threshold value,
and that the processing unit (50) determines in order to create the expiratory force value
- how high the signal energy is in a first frequency range, particularly between 10/min and 40/min, and
- how high the signal energy is in a second frequency range, particularly between 360/min and 900/min, and
that the processing unit (50) calculates the quotients from the energy in the first frequency range and the energy in the second frequency range, and uses this as the expiratory force value.

14. The device according to one of the preceding claims, **characterised in that** the processing unit (50)
a) creates a classification signal (A(t)), which contains for each audio segment (S_{Ai}) respectively at least one classification result stating the type and strength of the relevant noise in the time range of the respective audio segment (S_{Ai}),
b) respectively merges a number of consecutive audio segments (S_{Ai}) with further audio segments (S_{Ai}), particularly with a duration of 5 to 10 seconds, which preferably incorporate the same time ranges as the segments (S₁, ..., S_{q}),
c) carries out using the classification results contained within a further audio segment (S_{Ai}) an averaging and assigns the further audio segment (S_{Ai}) to the respective average value,
d) creates a further classification signal (B(t)) by means of interpolation of the average values of the further audio segments (S_{Ai}),
e) searches in the further classification signal B(t) and in the depth of breath for signal T(t) time points, at which strong changes occur in both signals (T(t), B(t)), and classifies the such identified time points as more relevant, the stronger the change of the respective signal at the respective time point is, wherein it assigns a relevant value relating to this respectively to these time points, and
f) detects those time points as the starting points (R_{S}) or end points (R_{E}) of a respiratory arrest, for which the amount of the relevance value is above the amount of a threshold value, wherein it particularly
- forms the threshold value by generating an average value of the reference measure over a time range, particularly before and/or after the comparison point with the reference measure and defining the threshold value in the range between 120% and 200% of this average value.

15. The device according to one of the preceding claims, **characterised in that** the processing unit (50)
- calculates a time range or number of time ranges, in which the change of the height profile (H), particularly on average, does not exceed a particularly threshold value, and
- to define the progress of the depth of breath only uses consecutively following recording times from the same time range, and
- where several time ranges exist carries out separately the determination of the progress of the depth of breath for a number of calculated time ranges.

## Revendications

1. Procédé de détermination de la variation temporelle de la profondeur respiratoire (T) d'une personne, en particulier endormie, une unité de détection (20) orientée vers la personne (1) crée en continu un profil de hauteur (H) de la personne (1) à des moments d'enregistrement séquentiels (t₁, ..., tₚ),
a) un certain nombre d'au moins deux points dans l'espace étant déterminés dans le profil de hauteur (H), qui se situent sur la surface de la personne (1) ou sur la surface d'un objet se trouvant sur la personne (1) ou à proximité de celle-ci,
b) pour chacun des moments d'enregistrement (t₁, ..., tₚ) le profil de hauteur (H) respectif étant enregistré dans une structure des données et tenu à disposition,
c) un certain nombre de profils de hauteur (H) enregistrés aux moments d'enregistrement séquentiels (t₁, ..., tₚ), en particulier dans une plage temporelle comprise entre 3 et 20 secondes, étant regroupé en un segment (S₁, ..., S_{q}),
d) une zone, qui indique la zone du ventre ou de la poitrine de la personne en fonction de chaque point de référence ou chaque zone de référence (21) étant sélectionnée comme zone d'observation (22),
e) pour chaque profil de hauteur (H) à l'intérieur du segment (S₁, ..., S_{q}), la moyenne des distances entre les points du profil de hauteur (H) se trouvant dans la zone d'observation (22) et un point de référence ou un objet de référence étant déterminée respectivement séparément, et pour le segment (S₁, ..., S_{q}) un signal (sᵢ) étant déterminé, auquel est associée au moment d'enregistrement respectif (t₁, ..., tₚ) du profil de hauteurs (H) la moyenne déterminée pour ledit profil de hauteur (H), et
f) sur la base du signal déterminé (sᵢ), en particulier sur la base sur l'amplitude de ce signal, une ou plusieurs valeurs caractérisant la variation temporelle (T) de la profondeur respiratoire est/sont déterminées, **caractérisé en ce que**
g) simultanément parallèlement à l'enregistrement de la distance entre la personne et l'unité de détection (20) le son émis par la personne est déterminé et tenu à disposition sous la forme d'un signal audio (sₐ) à l'aide d'un certain nombre de valeurs d'échantillonnage audio,
h) le signal audio (sₐ) est divisé en un certain nombre de segments audio (S_{A1}, ..., S_{Ar}), en particulier ayant une longueur comprise entre 100 ms et 1 seconde, et on examine pour chacun des segments audio (S_{A1}, ..., Sₐᵣ) si ce dernier contient des bruits respiratoires humains ou d'autres bruits, et pour chaque segment audio (S_{A1}, ..., S_{Ar}) un résultat de classification est tenu respectivement à disposition,
i) les segments audio enregistrés simultanément (S_{A1}, ..., Sₐᵣ) et les segments (S₁, ..., S_{q}) sont mutuellement associés, et
j) on recherche des segments audio (S_{A1}, ..., Sₐᵣ) ou des segments (S₁, ..., S_{q}) présentant des profondeurs respiratoires faibles ou des bruits respiratoires manquants et les segments (S₁, ..., S_{q}) associés à un tel segment audio (S_{A1}, ..., Sₐᵣ) ou segment (S₁, ..., S_{q}) ou les segments audio (S_{A1}, ..., Sₐᵣ) sont également examinés pour déterminer la présence de profondeurs respiratoires faibles ou de bruits respiratoires manquants, et
k) dans le cas où dans des segments associés mutuellement (S₁, ..., S_{q}) et dans des segments audio (S₁, ..., S_{q}) de faibles profondeurs respiratoires et des bruits respiratoires manquants ont été détectés, l'absence de respiration de la personne est constatée.

2. Procédé de détermination de la variation temporelle de la profondeur respiratoire (T) d'une personne, en particulier endormie, une unité de détection (20) orientée vers la personne (1) crée en continu un profil de hauteur (H) de la personne (1) à des moments d'enregistrement séquentiels (t₁, ..., tₚ),
a) le profil de hauteur (H) présentant un certain nombre d'au moins deux valeurs de distance (d₁, ..., dₙ) servant à déterminer respectivement un point dans l'espace, chacune des valeurs de distance indiquant respectivement la distance entre le point d'intersection d'un rayon partant en particulier de l'unité de détection, déterminé au préalable par rapport à l'unité de détection, et la surface de la personne (1) ou entre la surface d'un objet se trouvant sur la personne (1) ou à proximité de celle-ci et un point de référence ou un plan de référence (21)
b) pour chacun des moments d'enregistrement (t₁, ..., tₚ) une structure des données respective est créée qui contient le profil de hauteur (H) respectif, l'ensemble des structures des données ainsi créées présentant respectivement la même taille et présentant respectivement des positions de mémoire pour chacune des valeurs de distance du profil de hauteur (H),
c) un certain nombre de profils de hauteur (H) enregistrés aux moments d'enregistrement séquentiels (t₁, ..., tₚ), en particulier dans une plage temporelle comprise entre 3 et 20 secondes, étant regroupé en un segment (S₁, ..., S_{q}),
d) un certain nombre de positions de mémoire de la structure des données, dans lesquelles les valeurs de distance (d₁, ..., dₙ) sont enregistrées, qui indiquent la distance entre la zone du ventre ou la zone de la poitrine de la personne et chaque point de référence ou chaque zone de référence (21), étant sélectionné comme zone d'observation (22),
e) pour chaque profil de hauteur (H) dans un segment (S₁, ..., S_{q}), la moyenne des valeurs de distance (d₁, ..., dₙ) se trouvant à l'intérieur de la zone d'observation (22) étant déterminée respectivement séparément, et pour le segment (S₁, ..., S_{q}) un signal (sᵢ) étant déterminé, auquel est associée au moment d'enregistrement respectif (t₁, ..., tₚ) du profil de hauteur (H) la moyenne déterminée pour ledit profil de hauteur (H), et
f) sur la base du signal déterminé (sᵢ), en particulier sur la base sur l'amplitude de ce signal, une ou plusieurs valeurs caractérisant la variation temporelle (T) de la profondeur respiratoire est/sont déterminées, **caractérisé en ce que**
g) simultanément parallèlement à l'enregistrement de la distance entre la personne et l'unité de détection (20) le son émis par la personne est déterminé et tenu à disposition sous la forme d'un signal audio (sₐ) à l'aide d'un certain nombre de valeurs d'échantillonnage audio,
h) le signal audio (sₐ) est divisé en un certain nombre de segments audio (S_{A1}, ..., S_{Ar}), en particulier ayant une longueur comprise entre 100 ms et 1 seconde, et on examine pour chacun des segments audio (S_{A1}, ..., Sₐᵣ) si ce dernier contient des bruits respiratoires humains ou d'autres bruits, et pour chaque segment audio (S_{A1}, ..., S_{Ar}) un résultat de classification est tenu respectivement à disposition,
i) les segments audio enregistrés simultanément (S_{A1}, ..., Sₐᵣ) et les segments (S₁, ..., S_{q}) sont mutuellement associés, et
j) on recherche des segments audio (S_{A1}, ..., Sₐᵣ) ou des segments (S₁, ..., S_{q}) présentant des profondeurs respiratoires faibles ou des bruits respiratoires manquants et les segments (S₁, ..., S_{q}) associés à un tel segment audio (S_{A1}, ..., Sₐᵣ) ou segment (S₁, ..., S_{q}) ou les segments audio (S_{A1}, ..., Sₐᵣ) sont également examinés pour déterminer la présence de profondeurs respiratoires faibles ou de bruits respiratoires manquants, et
k) dans le cas où dans des segments associés mutuellement (S₁, ..., S_{q}) et dans des segments audio (S₁, ..., S_{q}) des faibles profondeurs respiratoires et des bruits respiratoires manquants ont été respectivement détectés, l'absence de respiration de la personne est constatée.

3. Procédé selon une des revendications précédentes, **caractérisé en ce**
a) **que** pour un certain nombre de segments (S₁, ..., S_{q}) se chevauchant ou ne se chevauchant pas, la profondeur respiratoire (T) est déterminée respectivement séparément et/ou
b) **que** la zone d'observation (22) pour chaque segment (S₁, ..., S_{q}) est déterminée séparément, en particulier sur la base de la zone d'observation (22) déterminée pour le segment respectif précédent (S₁, ..., S_{q}).

4. Procédé selon une des revendications précédentes, **caractérisé en ce que**
- le profil de hauteur (H) est **caractérisé par** une structure des données matricielle bidimensionnelle comprenant un certain nombre de lignes et de colonnes,
- un certain nombre de positions disposées sous forme de quadrillage dans des lignes et des colonnes est prédéfini, sur lesquelles respectivement les valeurs de distance (d₁, ..., dₙ) du profil de hauteur (H) sont déterminées,
- les valeurs de distance sont définies en particulier :
a) le long des rayons prédéfinis qui partent d'une unité de détection, ou
b) comme les distances normales entre le point d'intersection respectif du rayon avec la surface par rapport à un plan de référence (21) à l'aide de la distance mesurée et de l'angle de mesure respectif utilisé,
- de préférence les angles de mesure des différents rayons étant sélectionnés de telle sorte que lors de la rencontre des rayons sur un plan qui sont parallèles au plan de référence (21), permettent d'obtenir une disposition en forme de quadrillage,
- la structure des données matricielle présente un quadrillage de même taille et même structure, et
- la structure des données matricielle est créée par le fait que les valeurs de distance (d₁, ..., dₙ) enregistrées aux positions respectives sont stockées à des positions de mémoire dans la structure des données matricielle correspondantes aux positions dans le quadrillage et sont tenues à disposition, en particulier
a) la structure des données matricielle étant remplacée après sa création dans ses dimensions par une structure des données matricielle, pour les zones d'image ne se chevauchant pas, rectangulaires, en particulier recouvrant toute la structure des données matricielle, de taille respective identique dans la structure des données matricielle, une valeur de distance moyenne (d') étant respectivement déterminée et cette valeur de distance moyenne (d') étant associée au point d'image correspondant de la structure des données matricielle réduite par rapport à sa position, ou
b) la structure des données matricielle après sa création dans ses dimensions étant créée par une structure des données matricielle, dont la résolution spatiale est réduite par regroupement de plusieurs entrées de la structure des données matricielle en une entrée de la structure de données matricielle,
en particulier seules certaines valeurs de mesure de distance (d) étant utilisées pour la formation de la structure des données matricielle et les valeurs de mesure de distance restantes étant rejetées,
de préférence les paramètres (a, b) étant définies comme des valeurs entières et la structure des données matricielle réduite étant définie selon Hᵣ(x,y,t)=H(ax,by,t), ou
c) la structure des données matricielle après sa création dans ses dimensions étant remplacée par une structure des données matricielle réduite, pour des zones de la structure des données matricielle, une valeur de distance (d'), en particulier moyenne étant respectivement déterminée et cette valeur de distance (d') étant associée au point d'image de la structure des données matricielle correspondant par rapport à sa position.

5. Procédé selon une des revendications précédentes, **caractérisé en ce que** la zone d'observation (22) est fixée
a) pour l'ensemble des points du profil de hauteur (H), en particulier pour l'ensemble des entrées de la structure des données matricielle ou du nuage de points, par la création séparée d'un signal temporel pour l'intervalle temporel qui indique la variation temporelle du profil de hauteur (H) dans chaque point, et à partir dudit signal temporel une valeur d'intensité respiratoire caractérisant la profondeur respiratoire est respectivement dérivée, attribuée au point respectif du profil de hauteur,
b) par le fait qu'une zone ou plusieurs zones (22a, 22b) comprenant respectivement des points connexes du profil de hauteur (H), dont la valeur de force respiratoire respective se situe au-dessus d'une valeur seuil inférieure ou dans un intervalle prédéfini, est ou sont sélectionnée(s) en tant que zone d'observation (22), en particulier
- par la sélection des zones connexes (22a, 22b) des points en tant que zone d'observation, dont la taille dépasse un certain nombre prédéfini de points
- en particulier une zone étant ensuite considérée comme connexe, lorsque chaque point de la zone partant d'un autre point à l'intérieur de ladite zone peut être atteint par des pixels adjacents respectifs,
- de préférence des points du profil de hauteur (H) étant ensuite définis comme adjacents,
- lorsqu'ils sont déterminés par des entrées adjacentes de la structure des données matricielle ou
- lorsqu'ils ou leur projection sur un plan horizontal dans l'espace présente(nt) une distance inférieure à une valeur de seuil,
et pour la création de la valeur de force respiratoire par détermination de la puissance de l'énergie de signal dans une première gamme de fréquences, en particulier entre 10/min et 40/min, et par détermination de la puissance de l'énergie de signal dans une seconde gamme de fréquences, en particulier entre 360/min et 900/min, et un quotient est déterminé à partir de l'énergie de signal dans la première gamme de fréquences et de l'énergie de signal dans la seconde gamme de fréquence et utilisé comme valeur de force respiratoire.

6. Procédé selon une des revendications précédentes, **caractérisé en ce**
a) **qu'**un signal de classification (A(t)) est créé qui pour chaque segment audio (S_{Ai}) contient respectivement au moins un résultat de classification qui indique le type et l'intensité du bruit respectif dans la zone temporelle du segment audio respectif (S_{Ai}),
b) **que** respectivement un certain nombre de segments audio successifs (S_{Ai}) sont regroupés en d'autres segments audio (S_{Ai}), en particulier ayant une durée comprise entre 5 et 10 secondes qui de préférence comprennent les mêmes zones temporelles que les segments (S₁, ..., S_{q}),
c) **qu'**au moyen des résultats de classifications obtenus dans un autre segment audio (S_{Ai}) on procède à un moyennage et l'autre segment audio (S_{Ai}) est attribué à la moyenne respective,
d) **qu'**un autre signal de classification (B(t)) est créé par interpolation de la moyenne de l'autre segment audio (S_{Ai}),
e) **qu'**on recherche des points temporels dans l'autre signal de classification B(t) ainsi que dans le signal de profondeur respiratoire T(t) dans lesquels de fortes modifications se produisent dans les deux signaux (T(t), B(t)),
f) **que** plus la modification de chaque signal au moment respectif est importante, plus les moments ainsi détectés seront d'autant pertinents,
g) **qu'**une valeur de pertinence respective est attribuée à ces moments, et
h) **que** ces moments sont détectés comme des points de départ (R_{S}) ou des points d'extrémité (R_{E}) d'un arrêt respiratoire pour lesquels la valeur de pertinence (REL) se situe au-dessus d'une valeur seuil, en particulier
- la valeur seuil étant formée par le fait que sur une zone temporelle, en particulier avant et/ou après le moment de comparaison avec la mesure de référence se forme une moyenne de la mesure de référence et la valeur seuil est définie entre 120 % et 200 % de ladite moyenne.

7. Procédé selon une des revendications précédentes, **caractérisé en ce**
- **qu'**une zone temporelle ou plusieurs zones temporelles sont déterminées dans lesquelles la modification du profil de hauteur (H), en particulier au centre, ne dépasse pas une valeur seuil prédéfini, et
- **que** pour la détermination de la variation de la profondeur respiratoire seuls des moments d'enregistrement séquentiels provenant de la même zone temporelle sont utilisés, et
- **que** si nécessaire en présence de plusieurs zones temporelles la détermination de la variation respiratoire s'effectue séparément pour plusieurs zones temporelles déterminées.

8. Support d'informations sur lequel est stocké un programme d'exécution d'un procédé selon l'une quelconque des revendications précédentes dans un dispositif selon l'une des revendications 9 à 15.

9. Dispositif de détermination de la variation temporelle de la profondeur respiratoire (T) d'une personne, en particulier endormie, comprenant
- une unité de détection (20) orientable vers un endroit pour dormir, en particulier un lit (10) qui crée en continu respectivement un profil de hauteur (H) de la personne (1) à des moments d'enregistrement séquentiels (t₁, ..., tₚ), dans le profil de hauteur (H) étant déterminé un certain nombre d'au moins deux points dans l'espace, qui se trouvent sur la surface de la personne (1) ou sur la surface d'un objet se trouvant sur la personne (1) ou à proximité de celle-ci,
ainsi qu'
- une unité de traitement (50) qui
a) pour chacun des moments d'enregistrement (t₁, ..., tₚ) stocke et tient à disposition le profil de hauteur (H) respectif dans une structure des données,
b) regroupe un certain nombre de profils de hauteur (H) enregistrés aux moments d'enregistrement séquentiels (t₁, ..., tₚ), en particulier dans une plage temporelle comprise entre 3 et 20 secondes, en un segment (S₁, ..., S_{q}),
c) sélectionne une zone, qui indique la zone du ventre ou de la poitrine en fonction de chaque point de référence ou chaque zone de référence (21) comme zone d'observation (22),
d) détermine pour chaque profil de hauteur (H) dans le segment (S₁, ..., S_{q}) la moyenne des distances entre des points du profil de hauteur (H) se trouvant dans la zone d'observation (22) et un point de référence ou un objet de référence, et pour le segment (S₁, ..., S_{q}) un signal (sᵢ) est déterminé, auquel au moment d'enregistrement respectif (t₁, ..., tₚ) du profil de hauteur (H) est associée la moyenne déterminée pour ce profil de hauteur (H), et
e) détermine en particulier ensuite sur la base de ce signal (sᵢ) ou son amplitude une valeur caractérisant la profondeur respiratoire (T), **caractérisé en ce qu'**un microphone (40) est monté en amont de l'unité de traitement (50), qui simultanément parallèlement à l'enregistrement de la distance de la personne tient à disposition le son émis par la personne (1) sous forme d'un signal audio (sₐ) à sa sortie et ce signal audio est amené à l'unité de traitement et **en ce que** l'unité de traitement
f) divise le signal audio (sₐ) en un certain nombre de segments audio (S_{A1}, ..., S_{Ar}), en particulier ayant une longueur comprise entre 100 ms et 1 seconde, et examine pour chacun des segments audio (S_{A1}, ..., Sₐᵣ) si dans ce dernier on peut entendre des bruits respiratoires humains ou d'autres bruits, et pour chaque segment audio (S_{A1}, ..., S_{Ar}) un résultat de classification est tenu respectivement à disposition,
g) associe les uns aux autres les segments audio enregistrés simultanément (S_{A1}, ..., Sₐᵣ) et les segments (S₁, ..., S_{q}), et
h) recherche des segments audio (S_{A1}, ..., Sₐᵣ) ou des segments (S₁, ..., S_{q}) présentant des profondeurs respiratoires faibles ou des bruits respiratoires manquants et examine également les segments (S₁, ..., S_{q}) associés à un tel segment audio (S_{A1}, ..., Sₐᵣ) ou segment (S₁, ..., S_{q}) ou segments audio (S_{A1}, ..., Sₐᵣ) pour déterminer la présence de profondeurs respiratoires faibles ou de bruits respiratoires manquants, et
i) dans le cas où dans des segments associés mutuellement (S₁, ..., S_{q}) et dans des segments audio (S₁, ..., S_{q}) de faibles profondeurs respiratoires et des bruits respiratoires manquants ont été respectivement détectés, constate l'absence de respiration de la personne.

10. Dispositif de détermination de la variation temporelle de la profondeur respiratoire (T) d'une personne, en particulier endormie, comprenant
- une unité de détection (20) orientable vers un endroit pour dormir, en particulier un lit (10), qui crée en continu à des moments d'enregistrement séquentiels (t₁, ..., tₚ) un profil de hauteur (H) de la personne (1), le profil de hauteur (H) présentant un certain nombre d'au moins deux valeurs de distance (d₁, ..., dₙ) servant à déterminer respectivement un point dans l'espace, chacune des valeurs de distance indiquant respectivement la distance du point d'intersection d'un rayon partant en particulier de l'unité de détection, défini au préalable par rapport à l'unité de détection, à la surface de la personne (1) ou la surface d'un objet se trouvant sur la personne (1) ou à proximité de celle-ci et un point de référence ou un plan de référence (21),
ainsi qu'
- une unité de traitement (50) qui
a) pour chacun des moments d'enregistrement (t₁, ..., tₚ) produit respectivement une structure des données qui contient le profil de hauteur (H) respectif, l'ensemble des structures des données ainsi créées présentant respectivement la même taille et présentant respectivement des positions de mémoire pour chacune des valeurs de distance du profil de hauteur (H),
b) regroupe un certain nombre de profils de hauteur (H) enregistrés aux moments d'enregistrement séquentiels (t₁, ..., tₚ), en particulier dans une plage temporelle comprise entre 3 et 20 secondes, en un segment (S₁, ..., S_{q}),
c) sélectionne un certain nombre de positions de mémoire de la structure des données, dans lesquelles les valeurs de distance (d₁, ..., dₙ) sont stockées, qui indiquent la distance entre la zone du ventre ou la zone de la poitrine de la personne et chaque point de référence respectif ou chaque zone de référence respective (21), comme zone d'observation (22),
d) détermine pour chaque profil de hauteur (H) dans le segment (S₁, ..., S_{q}), la moyenne des distances (d₁, ..., dₙ) se trouvant dans la zone d'observation (22) et détermine pour le segment (S₁, ..., S_{q}) un signal (sᵢ), auquel est associé au moment d'enregistrement respectif (t₁, ..., tₚ) du profil de hauteur (H) la moyenne déterminée pour ce profil de hauteur (H), et
e) détermine en particulier ensuite sur la base de ce signal (sᵢ) ou son amplitude une valeur caractérisant la profondeur respiratoire (T), **caractérisé en ce qu'**un microphone (40) est monté en amont de l'unité de traitement (50), qui simultanément parallèlement à l'enregistrement de la distance de la personne tient à disposition le son émis par la personne (1) sous forme d'un signal audio (sₐ) à sa sortie et ce signal audio est amené à l'unité de traitement et **en ce que** l'unité de traitement
f) divise le signal audio (sₐ) en un certain nombre de segments audio (S_{A1}, ..., S_{Ar}), en particulier ayant une longueur comprise entre 100 ms et 1 seconde, et examine pour chacun des segments audio (S_{A1}, ..., Sₐᵣ) si dans ce dernier on peut entendre des bruits respiratoires humains ou d'autres bruits, et tient respectivement à disposition pour chaque segment audio (S_{A1}, ..., S_{Ar}) un résultat de classification,
g) associe les uns aux autres les segments audio enregistrés simultanément (S_{A1}, ..., Sₐᵣ) et les segments (S₁, ..., S_{q}), et
h) recherche des segments audio (S_{A1}, ..., Sₐᵣ) ou des segments (S₁, ..., S_{q}) présentant des profondeurs respiratoires faibles ou des bruits respiratoires manquants et examine également les segments (S₁, ..., S_{q}) associés à un tel segment audio (S_{A1}, ..., Sₐᵣ) ou segment (S₁, ..., S_{q}) ou segments audio (S_{A1}, ..., Sₐᵣ) pour déterminer la présence de profondeurs respiratoires faibles ou de bruits respiratoires manquants, et
i) dans le cas où dans des segments associés mutuellement (S₁, ..., S_{q}) et dans des segments audio (S₁, ..., S_{q}) de faibles profondeurs respiratoires et des bruits respiratoires manquants ont été respectivement détectés, constate l'absence de respiration de la personne.

11. Dispositif selon l'une des revendications précédentes, **caractérisé en ce que** l'unité de traitement (50)
a) pour un certain nombre de segments (S₁, ..., S_{q}) se chevauchant ou ne se chevauchant pas, détermine la profondeur respiratoire (T) et/ou
b) détermine séparément la zone d'observation (22) pour chaque segment (S₁, ..., S_{q}), en particulier sur la base de la zone d'observation (22) déterminée pour le segment précédent respectif (S₁, ..., S_{q}).

12. Dispositif selon l'une des revendications précédentes, **caractérisé en ce que** l'unité de traitement (50)
- caractérise le profil de hauteur (H) par une structure des données matricielle bidimensionnelle comprenant un certain nombre de lignes et de colonnes,
- définit un certain nombre de positions disposées sous forme de quadrillage dans les lignes et les colonnes, sur lesquelles chacune des valeurs de distance (d₁, ..., dₙ) du profil de hauteurs (H) est déterminée,
- définit les valeurs de distance en particulier
a) en tant que valeurs de distance le long des rayons prédéfinis qui partent d'une unité de détection, ou
b) comme les distances normales entre le point d'intersection respectif du rayon avec la surface par rapport à un plan de référence (21) à l'aide de la distance mesurée et de l'angle de mesure respectif utilisé,
- elle sélectionne de préférence l'angle de mesure des différents rayons de telle sorte que lors de la rencontre des rayons sur un plan qui sont parallèles au plan de référence (21) permettent de former une disposition en forme de quadrillage,
- la structure des données matricielle présente un quadrillage de même taille et même structure, et
- crée la structure des données matricielle par le fait qu'elle stocke les valeurs de distance (d₁, ..., dₙ) enregistrées à chacune des positions, dans des positions de mémoire dans la structure des données correspondant aux positions dans le quadrillage et les tient à disposition, et **en ce que**
a) l'unité de traitement (50) remplace la structure des données matricielle après sa création dans ses dimensions par une structure des données matricielle réduite,
elle détermine respectivement pour en particulier des zones d'image ne se chevauchant pas et recouvrant toute la structure des données matricielle de taille respectivement identique dans la structure des données matricielle, une valeur de distance moyenne (d') et associe cette valeur de distance moyenne (d') à un point d'image de la structure des données matricielle réduite, correspondant par rapport à sa position, ou
b) l'unité de données (50) remplace la structure des données matricielle après sa création dans ses dimensions par une structure des données matricielle réduite, dont la résolution spatiale est réduite par regroupement de plusieurs entrées de la structure des données matricielles en une entrée de la structure de données matricielle,
elle utilise en particulier seules certaine valeurs de mesure de distance (d) pour la formation de la structure des données matricielle réduite et rejette les valeurs de mesure de distance restantes, elle détermine de préférence les paramètres (a, b) en tant que valeurs entières et définit la structure des données matricielle réduite selon Hᵣ(x,y,t)=H(ax,by,t), ou
c) l'unité de traitement (50) remplace la structure des données matricielle après sa création dans ses dimensions par une structure des données matricielle réduite, elle détermine respectivement pour des zones de la structure des données matricielle, une valeur de distance (d'), en particulier moyenne et associe cette valeur de distance (d') au point d'image de la structure des données matricielle correspondant par rapport à sa position.

13. Dispositif selon une des revendications 9 à 12, **caractérisé en ce que** l'unité de traitement (50) définit la zone d'observation (22) du fait qu'elle crée séparément
a) pour l'ensemble des points du profil de hauteur (H), en particulier pour l'ensemble des entrées de la structure des données matricielle ou des nuages de points, un signal temporel pour l'intervalle temporel qui indique la variation temporelle du profil de hauteur (H) dans chaque point, et à partir de ce signal temporel, fait dériver respectivement une valeur de force respiratoire caractérisant la profondeur respiratoire et l'attribue au point respectif du profil de hauteur,
b) sélectionne une zone ou plusieurs zones (22a, 22b) comprenant respectivement des points connexes du profil de hauteur (H), dont la valeur de force respiratoire respective se situe au-dessus d'une valeur seuil inférieure ou dans un intervalle prédéfini, en tant que zone d'observation (22), en particulier
- en sélectionnant des zones connexes (22a, 22b) des points en tant que zone d'observation, dont la taille dépasse un certain nombre prédéfini de points,
- en particulier une zone étant ensuite considérée comme connexe, lorsque chaque point de la zone en partant d'un autre point à l'intérieur de ladite zone peut être atteint par des pixels adjacents respectifs,
- de préférence des points du profil de hauteur (H) étant ensuite considérés comme adjacents,
- lorsqu'ils sont déterminés par des entrées adjacentes de la structure des données matricielle ou
- lorsqu'ils ou leur projection sur un plan horizontal dans l'espace présente(nt) une distance inférieure à une valeur de seuil,
et que l'unité de traitement (50) détermine pour la création de la valeur de force respiratoire,
- la puissance de l'énergie de signal dans une première gamme de fréquences, en particulier entre 10/min et 40/min, et
- la puissance de l'énergie de signal dans une seconde gamme de fréquences, en particulier entre 360/min et 900/min, et
**en ce que** l'unité de traitement (50) détermine les quotients à partir de l'énergie dans la première gamme de fréquences et de l'énergie dans la seconde gamme de fréquences, et les utilise en tant que valeur de force respiratoire.

14. Dispositif selon l'une des revendications précédentes, **caractérisé en ce que** l'unité de traitement (50)
a) produit un signal de classification (A(t)) qui pour chaque segment audio (S_{Ai}) contient respectivement au moins un résultat de classification qui indique le type et l'intensité du bruit respectif dans la zone temporelle du segment audio respectif (S_{Ai}),
b) regroupe respectivement un certain nombre de segments audio séquentiels (S_{Ai}) en d'autres segments audio (S_{Ai}), en particulier ayant une durée comprise entre 5 et 10 secondes, qui de préférence comprennent les mêmes zones temporelles que les segments (S₁, ..., S_{q}),
c) procède au moyen des résultats de classifications obtenus dans un autre segment audio (S_{Ai}) à un moyennage et attribue à l'autre segment audio (S_{Ai}) la valeur moyenne respective,
d) produit un autre signal de classification (B(t)) par interpolation des moyennes de l'autre segment audio (S_{Ai}),
e) recherche dans l'autre signal de classification B(t) ainsi que dans le signal de profondeur respiratoire T(t), des moments auxquels dans les deux signaux (T(t), B(t)) se produisent de fortes modifications et plus la modification du signal respectif au moment respectif est importante, plus les moments ainsi détectés sont classés comme pertinents, l'unité de traitement attribuant respectivement ces moments à une mesure de pertinence, et
f) détecte ces moments en tant que points de départ (R_{S}) ou points d'extrémité (R_{E}) d'un arrêt respiratoire pour lesquels la valeur pertinente (REL) est supérieure à une valeur seuil, l'unité de traitement formant en particulier
- la valeur seuil en formant par une zone temporelle, en particulier avant et/ou après le moment temporel de comparaison avec la mesure de référence, une moyenne de la mesure de référence et en définissant la valeur seuil entre 120 % et 200 % de ladite moyenne.

15. Dispositif selon l'une des revendications précédentes, **caractérisé en ce que** l'unité de traitement (50)
- détermine une zone temporelle ou plusieurs zones temporelles dans lesquelles la modification du profil de hauteur (H), en particulier au centre, ne dépasse pas une valeur seuil prédéfinie, et
- utilise pour la détermination de la variation de la profondeur respiratoire uniquement des moments d'enregistrement séquentiels provenant de la même zone temporelle, et
- si nécessaire en présence de plusieurs zones temporelles détermine séparément la variation de la profondeur respiratoire pour plusieurs zones temporelles déterminées.
